# EUROPÄISCHE PATENTSCHRIFT

(11) **EP 3 082 960 B1**
(45) Veröffentlichungstag und Bekanntmachung des Hinweises auf die Patenterteilung: **06.03.2019**
(21) Anmeldenummer: 14806243.3
(22) Anmeldetag: 02.12.2014
(51) Int. Cl.: A61Q 5/04, A61Q 9/04, A61K 8/34, A61K 8/44, A61K 8/46, A45D 2/02, A45D 2/12, A61K 8/37, A61K 8/86, A45D 7/04

(54) **GERUCHSREDUZIERTE MITTEL ZUM GLÄTTEN ODER PERMANENTEN UMFORMEN VON HAAREN ODER ZUR DEPILATION**
ODOR-REDUCED AGENT FOR SMOOTHING OR PERMANENTLY SHAPING HAIR OR FOR DEPILATION
AGENT RÉDUCTEUR D'ODEUR POUR LE LISSAGE OU LA TRANSFORMATION PERMANENTE DES CHEVEUX OU POUR LA DÉPILATION

(30) Priorität: 17.12.2013 DE 102013226278
(43) Veröffentlichungstag der Anmeldung: 26.10.2016
(73) Patentinhaber: Henkel AG & Co. KGaA, 40589 Düsseldorf (DE)
(72) Erfinder: SCHETTIGER, Norbert, 40723 Hilden (DE); NEUBA, Constanze, 41516 Grevenbroich (DE); JANSSEN, Frank, 51103 Köln (DE); MÜLLER, Burkhard, 40221 Düsseldorf (DE)
(86) Internationale Anmeldenummer: PCT/EP2014/076178
(87) Internationale Veröffentlichungsnummer: WO 2015/090929

(56) Entgegenhaltungen:
- JP-A- 2001 072 553
- JP-A- 2008 184 447
- JP-A- 2013 170 138
- US-A1- 2005 196 367

## Beschreibung

Gegenstand der vorliegenden Erfindung sind Mittel zum Glätten, permanenten Umformen oder zur Depilation von keratinischen Fasern, insbesondere menschlichen Haaren, die den bei Dauerwell-, Haarglättungs- und Depiliermitteln auftretenden intensiven und reizenden Geruch der schwefelhaltigen Reduktionsmittel (Thioglycolat, Cystein etc.) minimieren.

Die dauerhafte Verformung von Keratinfasern wird üblicherweise so durchgeführt, dass man die Faser mit Hilfe von mechanischen Verformungshilfsmitteln (Wickler, Papilloten) verformt und die Verformung durch geeignete Hilfsmittel festlegt. Vor und/oder nach dieser Verformung behandelt man die Faser mit der wässrigen Zubereitung einer Keratin reduzierenden Substanz und spült nach einer Einwirkungszeit mit Wasser oder einer wässrigen Lösung. In einem zweiten Schritt behandelt man dann die Faser mit der wässrigen Zubereitung eines Oxidationsmittels. Nach einer Einwirkungszeit wird auch dieses ausgespült und die Faser von den mechanischen Verformungshilfsmitteln (Wickler, Papilloten) befreit.

Die wässrige Zubereitung der Keratin reduzierenden Substanz ist üblicherweise alkalisch eingestellt, damit zum einen ein genügender Anteil der Thiolfunktionen deprotoniert vorliegt und zum anderen die Faser quillt und auf diese Weise ein tiefes Eindringen der Keratin reduzierenden Substanz in die Faser ermöglicht wird. Die keratinreduzierende Substanz spaltet einen Teil der Disulfid-Bindungen des Keratins zu -SH-Gruppen, so dass es zu einer Lockerung der Peptidvernetzung und infolge der Spannung der Faser durch die mechanische Verformung zu einer Neuorientierung des Keratingefüges kommt. Unter dem Einfluss des Oxidationsmittels werden erneut Disulfid-Bindungen geknüpft, und auf diese Weise wird das Keratingefüge in der vorgegebenen Verformung neu fixiert. Ein bekanntes derartiges Verfahren stellt die Dauerwell-Behandlung menschlicher Haare dar. Dieses kann sowohl zur Erzeugung von Locken und Wellen in glattem Haar als auch zur Glättung von gekräuselten oder ethnischen Haaren angewendet werden. Den vorstehend beschriebenen Reaktionsmechanismus zur Spaltung der Disulfid-Bindungen des Keratins zu -SH-Gruppen ohne nachfolgende Oxidation macht man sich auch bei der Depilation, der chemischen Haarentfernung von behaarter Haut, zu Nutze. Nach Einwirkung der Keratin reduzierenden Substanz(en) auf die behaarte Haut, z. B. an den Beinen, sind die Haare leicht abzuschaben, ohne dass eine die Haut reizende Rasur erfolgen muss.

Eine Verbindung gilt im Sinne der Erfindung als Keratin reduzierend, wenn eine Lösung von 50 mmol dieser Verbindung in 100 g Wasser bei 20°C und einem pH-Wert von pH 8 (eingestellt mit 2-Aminoethanol) Disulfid-Bindungen des Keratins zu -SH-Gruppen spaltet.

Übliche Keratin reduzierende Substanzen sind ausgewählt aus Thioglykolsäure und deren Salzen, und /oder Thiomilchsäure und deren Salzen und/oder Cystein und/oder Acetylcystein und/oder Cysteamin und deren Salzen sowie Mischungen derselben. Diese Substanzen weisen einen deutlichen und sehr unangenehmen schwefligen Geruch auf.

Um eine zufriedenstellende Keratin reduzierende Leistung zu entfalten, benötigen die erfindungsgemäßen Mittel bei der Anwendung in der Regel einen alkalischen oder nur schwach sauren pH-Wert. Insbesondere bei pH-Werten im Bereich von 7 bis 9,5 werden optimale Ergebnisse erzielt. Zur Einstellung dieser pH-Werte ist bis zum heutigen Zeitpunkt Ammoniak das Alkalisierungsmittel der Wahl. Mit Ammoniak lässt sich nicht nur der für die Farbstoffbildung notwendige pH-Bereich einstellen, sondern Ammoniak sorgt auch in stärkerem Maß als alle anderen bekannten Alkalisierungsmittel für eine Quellung der Haare. Gleichzeitig wirkt Ammoniak - ebenfalls in stärkerem Ausmaß als alle anderen handelsüblichen Alkalisierungsmittel - als Penetrations- bzw. Penetrationshilfsmittel. Die mit dem Einsatz von Ammoniak verbundenen anwendungstechnischen Vorteile sind so vielfältig, dass Ammoniak trotz seines unangenehmen, stechenden Geruchs in einer Vielzahl handelsüblicher Glätt-, Well- und Depiliermittel verwendet wird.

Eine prinzipielle Möglichkeit zur Reduktion des schwefligen Geruchs besteht im Zusatz von speziellen Parfümstoffen, welche den Geruch überdecken sollen. Parfümstoffe können jedoch unter den alkalischen Lagerbedingungen instabil sein, so dass die Gefahr besteht, dass die Duftstoffe während der Lagerung abgebaut oder strukturell verändert werden, was sich auch in einer unvorhersehbaren Änderung des Geruchs niederschlägt. Da entsprechende Veränderungen oftmals erst nach mehreren Monaten oder sogar Jahren wahrnehmbar werden, wird der Einsatz von neuen bzw. unbekannten Parfums als problematisch eingestuft.

Eine weitere prinzipielle Möglichkeit zur Reduktion des schwefligen Geruchs besteht in einer Optimierung der Formulierung. Hierbei gilt es, die Träger-Bestandteile der Formulierung so auszuwählen, dass diese einen optimalen Rückhalt der Keratin reduzierenden Substanzen in der Formulierung gewährleisten und auf diese Weise deren Geruch minimieren. Es ist jedoch ebenfalls bekannt, dass die Formulierung, die ggf. in ihr enthaltenen Fettstoffe, ihre Emulgatoren, Tenside und ihre Viskosität wesentlichen Einfluss auf die keratolytische Leistung nehmen. Bei der Modifikation der Formulierung muss eine Verschlechterung der keratolytischen Leistung daher auf jeden Fall vermieden werden.

Insbesondere die dauerhafte Geruchsminimierung über die gesamte Anwendungsdauer ist nur sehr schwierig zu erreichen. Die Zeitspanne, innerhalb der sich der Anwender in Kontakt mit dem Well-, Glätt- oder Depiliermittel befindet, reicht von der Auftragung des Mittels auf die Haare und den Zeitraum des Einwirkens bis zum Auswaschen der Formulierung. Bei üblichen Einwirkzeiten von 5-60 Minuten, bevorzugt 10-30 Minuten kann der gesamte Prozess bis zu 75 Minuten Zeit in Anspruch nehmen. Eine geruchliche Abdeckung der Keratin reduzierenden Substanzen, die über diesen gesamten Zeitraum wirksam ist, stellt die höchste Herausforderung dar. Gerade auf diesem Feld besteht noch starker Optimierungsbedarf, und eine optimale Möglichkeit zur dauerhaften Reduktion des Geruchs der Keratin reduzierenden Substanzen ist aus dem Stand der Technik bislang nicht bekannt.

JP 2001 072553 A beschäftigt sich unter anderem mit Dauerwellmitteln, die eine verbesserte Penetration des Haarverformenden Wirkstoffs in das Haar aufweisen, wobei diese Mittel eine möglichst geringe Hautreizung verursachen sollen. JP 2008 184447 A beschäftigt sich damit, den stechenden Geruch von Keratin-reduzierenden Zusammensetzungen für die permanente Haarverformung (Dauerwellen und Glättungsmittel) zu reduzieren.

Aufgabe der vorliegenden Erfindung war daher die Bereitstellung von Mitteln zum Glätten, permanenten Umformen oder zur Depilation von Haaren mit reduziertem schwefligen Geruch. Hierbei sollen die Mittel keine Einbußen in ihrer Keratin reduzierenden Leistung aufweisen.

Es war hierbei insbesondere die Aufgabe der vorliegenden Erfindung, über die gesamte Anwendungsdauer eine Reduktion des schwefligen Geruchs zu erzielen. Auch nach maximal zweistündigem Kontakt sollte die geruchliche Wahrnehmung der Keratin reduzierenden Substanzen immer noch wirksam minimiert sein.

Überraschenderweise hat sich im Zuge der zu dieser Erfindung führenden Arbeiten herausgestellt, dass es möglich ist, die geruchliche Wahrnehmung von Keratin reduzierenden Substanzen in Mitteln zum Glätten, permanenten Umformen oder zur Depilation von keratinischen Fasern wirksam über den gesamten Anwendungszeitraum zu minimieren, wenn den Mitteln neben der mindestens einen Keratin reduzierenden Verbindung und Wasser eine Kombination aus speziellen Fettalkoholen und speziellen Glycerylfettsäureestern zugesetzt wird.

Ein erster Gegenstand der vorliegenden Erfindung ist daher ein Mittel zum Glätten, permanenten Umformen oder zur Depilation von keratinischen Fasern, insbesondere menschlichen Haaren, enthaltend
(a) mindestens ein lineares Alkan-1-ol oder Alken-1-ol mit jeweils 20 bis 22 Kohlenstoffatomen, wobei das mindestens eine Alken-1-ol eine bis vier olefinische Doppelbindungen aufweist, in einer Gesamtmenge von 1,8 bis 10,0 Gew.-%, bezogen auf das Gewicht des Mittels, weiter
(b) mindestens einen Glycerylfettsäureester der allgemeinen Formel (I), 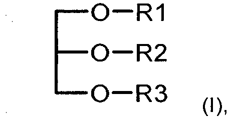 worin
   R1, R2 und R3 unabhängig voneinander für ein Wasserstoffatom oder eine Gruppierung der Formel (II) stehen, 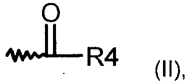 worin
   R4 für eine unverzweigte oder verzweigte, gesättigte oder ungesättigte C₁₁-C₂₇-Alkylgruppe steht,
   mit der Maßgabe, dass mindestens einer und maximal zwei der Reste ausgewählt aus R1, R2 und R3 für eine Gruppierung der Formel (II) stehen, weiterhin
(c) Wasser und
(d) mindestens eine Keratin reduzierende Verbindung, ausgewählt aus Thioglykolsäure und deren Salzen und / oder Thiomilchsäure und deren Salzen und/oder Cystein und/oder Acetylcystein und/oder Cysteamin und deren Salzen sowie Mischungen dieser Keratin reduzierenden Verbindungen,
dadurch gekennzeichnet, dass der mindestens eine Glvcervlfettsäureester (b) der allgemeinen Formel (I) in einer Gesamtmenge von 0,1 bis 0,8 Gew.-% - bezogen auf das Gewicht des Mittels - enthalten ist.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Als ersten erfindungswesentlichen Formulierungsbestandteil (a) enthalten die erfindungsgemäßen Mittel zum Glätten, permanenten Umformen oder zur Depilation von Keratinfasern mindestens ein lineares Alkan-1-ol oder Alken-1-ol mit jeweils 20 bis 22 Kohlenstoffatomen in einer Gesamtmenge von 1,8 bis 10,0 Gew.-%, bezogen auf das Gewicht des Mittels, wobei das mindestens eine Alken-1-ol eine bis vier olefinische Doppelbindungen aufweist.

Bevorzugte lineare Alkan-1-ol und Alken-1-ole mit jeweils 20 bis 22 Kohlenstoffatomen, wobei das mindestens eine Alken-1-ol eine bis vier olefinische Doppelbindungen aufweist, sind ausgewählt aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol), sowie aus Mischungen hiervon.

Daher ist es bevorzugt, wenn die erfindungsgemäßen Mittel einen oder mehrere lineare Alkan-1-ole oder Alken-1-ole mit jeweils 20 bis 22 Kohlenstoffatomen aus der Gruppe Arachylalkohol (Eicosan-1-ol), Gadoleylalkohol ((9*Z*)-Eicos-9-en-1-ol), Arachidonalkohol ((5Z,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol) sowie Mischungen hiervon enthalten.

Besonders bevorzugte erfindungsgemäße Mittel enthalten einen oder mehrere lineare Alkan-1-ole oder Alken-1-ole mit jeweils 20 bis 22 Kohlenstoffatomen, wobei das mindestens eine Alken-1-ol eine bis vier olefinische Doppelbindungen aufweist, bevorzugt mindestens ein Alkanol/Alkenol der vorgenannten Gruppe in einer Gesamtmenge von 2,0 bis 8,0 Gew.-%, bevorzugt von 2,0 bis 6,0 Gew.-% und besonders bevorzugt von 2,0 bis 4.0 Gew.-%, jeweils bezogen auf das Gewicht des Mittels.

In einer weiteren bevorzugten Ausführungsform ist ein erfindungsgemäßes Mittel daher dadurch gekennzeichnet, dass es einen oder mehrere lineare Alkan-1-ole oder Alken-1-ole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol) in einer Gesamtmenge von 1,8 bis 10,0 Gew.-%, bevorzugt von 2,0 bis 8,0 Gew.-%, weiter bevorzugt von 2,0 bis 6,0 Gew.-% und besonders bevorzugt von 2,0 bis 4,0 Gew.-%, jeweils bezogen auf das Gewicht des Mittels, enthält.

Als zweiten erfindungswesentlichen Bestandteil enthält das erfindungsgemäße Mittel mindestens einen Glycerylfettsäureester der allgemeinen Formel (I), 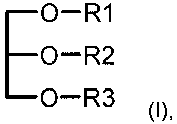 worin
R1, R2 und R3 unabhängig voneinander für ein Wasserstoffatom oder eine Gruppierung der Formel (II) stehen, 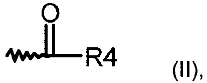 worin
R4 für eine unverzweigte oder verzweigte, gesättigte oder ungesättigte C₁₁-C₂₇-Alkylgruppe steht,
mit der Maßgabe, dass mindestens einer und maximal zwei der Reste ausgewählt aus R1, R2 und R3 für eine Gruppierung der Formel (II) stehen, in einer Gesamtmenge von 0,1 bis 0,8 Gew.-%, bezogen auf das Gewicht des Mittels.

Bevorzugt steht R4 für eine unverzweigte, gesättigte C₁₁-C₂₇-Alkylgruppe. Weiter bevorzugt steht R4 für eine unverzweigte, gesättigte C₁₃-C₂₃-Alkylgruppe. Besonders bevorzugt steht R4 für eine unverzweigte, gesättigte C₁₅-C₁₇-Alkylgruppe.

Die Glycerylfettsäureester (b) bewirken in Kombination mit den langkettigen Fettalkoholen (a) eine Reduktion des schwefligen Geruchs. Die Geruchsreduktion ist besonders ausgeprägt, wenn die Gesamtmenge an Glycerylfettsäureester(n) (b) und C20-C22-Fettalkohol(en) (a) in bestimmten Verhältnissen zueinander eingesetzt werden. Daher sind auch die Glycerylfettsäureester (b) bevorzugt in bestimmten Mengen im erfindungsgemäßen Mittel enthalten.

Besonders bevorzugt ist es, wenn im erfindungsgemäßen Mittel ein oder mehrere Glycerylfettsäureester (b) in einer Gesamtmenge von 0,2 bis 0,6 Gew.-%, weiter bevorzugt von 0,25 bis 0,6 Gew.-% und besonders bevorzugt von 0,3 bis 0,6 Gew.-% - bezogen auf das Gesamtgewicht des anwendungsbereiten Mittels - enthalten sind.

In einer weiteren besonders bevorzugten Ausführungsform ist ein erfindungsgemäßes Mittel dadurch gekennzeichnet, dass es als Glycerylfettsäureester (b) der allgemeinen Formel (I) mindestens eine Verbindung aus der Gruppe der Formeln (Ia) bis (Id) enthält.

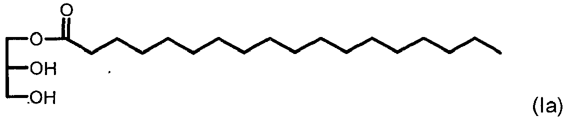

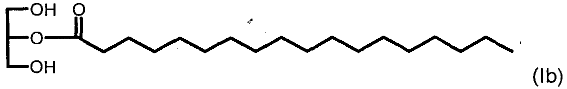

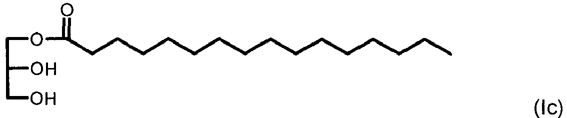

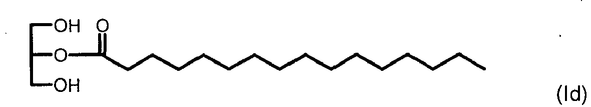

Die Verbindungen der Formeln (Ia) bis (Id) sind auch unter den Namen Glycerylmonostearat und Glycerylmonopalmitat bekannt.

In einer weiteren besonders bevorzugten Ausführungsform ist ein erfindungsgemäßes Mittel dadurch gekennzeichnet, dass es als Glycerylfettsäureester (b) der allgemeinen Formel (I) mindestens eine Verbindung aus der Gruppe der Formeln (Ie) bis (Ih) enthält.

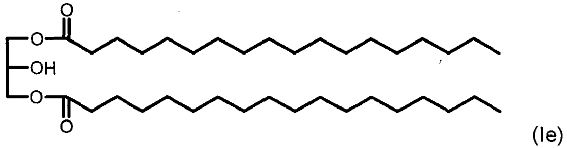

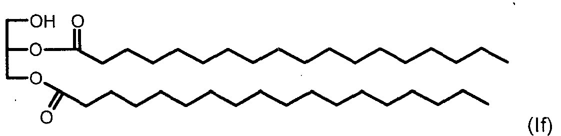

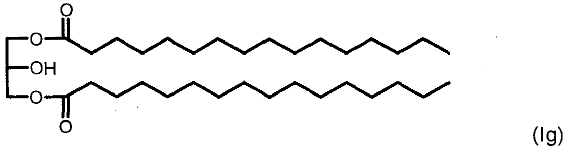

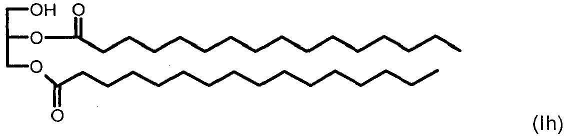

Die Verbindungen der Formel (Ie) bis (Ih) sind auch unter den Namen Glyceryldistearat und Glyceryldi palmitat bekannt.

In einer ganz besonders bevorzugten Ausführungsform ist ein erfindungsgemäßes Mittel dadurch gekennzeichnet, dass es als Glycerylfettsäureester (b) der allgemeinen Formel (I) mindestens eine Verbindung, ausgewählt aus den Verbindungen mit den Formeln (Ia) bis (Ih), enthält.

In einer weiteren ganz besonders bevorzugten Ausführungsform ist ein erfindungsgemäßes Mittel dadurch gekennzeichnet, dass es als Glycerylfettsäureester (b) der allgemeinen Formel (I) mindestens eine Verbindung, ausgewählt aus Glycerylmonostearat, Glyceryldistearat, Glycerylmonopalmitat und Glyceryldipalmitat sowie aus Mischungen hiervon enthält.

In einer weiteren ganz besonders bevorzugten Ausführungsform ist ein erfindungsgemäßes Mittel dadurch gekennzeichnet, dass es als Glycerylfettsäureester (b) der allgemeinen Formel (I) mindestens eine Verbindung, ausgewählt aus Glycerylmonostearat, Glyceryldistearat, Glycerylmonopalmitat und Glyceryldipalmitat sowie Mischungen hiervon in einer Gesamtmenge von 0,1 bis 0,8 Gew.-%, weiter bevorzugt von 0,2 bis 0,6 Gew.-%, weiter bevorzugt von 0,25 bis 0,6 Gew.-% und besonders bevorzugt von 0,3 bis 0,6 Gew.-% - bezogen auf das Gewicht des Mittels - enthält.

Die erfindungsgemäßen Mittel enthalten Wasser, bevorzugt in einer Menge von 50 - 80 Gew.-%, besonders bevorzugt 60 - 75 Gew.-%, jeweils bezogen auf das Gewicht des Mittels.

Die erfindungsgemäßen Mittel enthalten mindestens eine Keratin reduzierende Verbindung (d), ausgewählt aus Thioglykolsäure und deren Salzen und/oder Thiomilchsäure und deren Salzen und/oder Cystein und/oder Acetylcystein und/oder Cysteamin und deren Salzen sowie Mischungen dieser Keratin reduzierenden Verbindungen. Bevorzugte erfindungsgemäße Mittel sind dadurch gekennzeichnet, dass die mindestens eine Keratin reduzierende Verbindung (d) in einer Gesamtmenge von 0,1 bis 20 Gew.%, bevorzugt 1 bis 15 Gew.-%, besonders bevorzugt 3 bis 13 Gew.-%, jeweils bezogen auf das Gewicht des Mittels, enthalten ist. Außerordentlich bevorzugt sind Mittel, die Thioglykolsäure und deren Salze und/oder Thiomilchsäure und deren Salze in einer Gesamtmenge von 5 bis 20 Gew.%, bevorzugt 7 bis 15 Gew.-%, besonders bevorzugt 10 bis 13 Gew.-%, jeweils bezogen auf das Gewicht des Mittels, enthalten.

Wenn die vorgenannten Keratin reduzierenden Verbindungen Thioglykolsäure, Thiomilchsäure, Cystein, Acetylcystein und/oder Cysteamin als Salz vorliegen, kommen prinzipiell alle physiologisch verträglichen Kationen als Gegenionen in Frage. Insbesondere sind dies Metallkationen der physiologisch verträglichen Metalle aus den Gruppen Ia, Ib, IIa, IIb, IIIb, VIa oder VIII des Periodensystems der Elemente, sowie kationische organische Verbindungen mit quaterniertem Stickstoffatom. Letztere werden beispielsweise durch Protonierung primärer, sekundärer oder tertiärer organischer Amine mit einer Säure, gebildet. Darunter sind (C₂ bis C₆)-Alkanolammonium-Ionen bevorzugt. Beispiele dieser kationischen organischen (C₂ bis C₆)-Alkanolammonium-Ionen sind 2-Ammonioethanol und 2-Trimethylammonioethanol.

Unter dem Begriff (C₂ bis C₆)-Alkanolammonium-Ion sind erfindungsgemäß organische Ammoniumverbindungen zu verstehen, die zwei bis sechs Kohlenstoffatome besitzen, welche ein Kohlenstoffgerüst bilden, an das mindestens eine Ammoniogruppe (bevorzugt genau eine Ammoniogruppe) und mindestens eine Hydroxygruppe (wiederum bevorzugt genau eine Hydroxygruppe) bindet. Bevorzugt geeignete (C₂ bis C₆)-Alkanolammonium-Ionen sind 2-Ammonioethan-1-ol, 3-Ammoniopropan-1-ol, 4-Ammoniobutan-1-ol, 5-Ammoniopentan-1-ol, 1-Ammoniopropan-2-ol, 1-Ammoniobutan-2-ol, 1-Ammo-niopentan-2-ol, 1-Ammoniopentan-3-ol, 1-Ammoniopentan-4-ol, 3-Ammonio-2-methylpropan-1-ol, 1-Ammonio-2-methylpropan-2-ol, 3-Ammoniopropan-1,2-diol oder 2-Ammonio-2-methylpropan-1,3-diol.

Tragen die keratinreduzierenden Verbindungen ein Chiralitätszentum, wie beispielsweise Cystein, sind jegliche Stereoisomere der besagten chiralen Derivate umfasst. Bevorzugt handelt es sich um die L-Form.

Im Zuge der zu dieser Erfindung führenden Arbeiten hat sich weiterhin gezeigt, dass die erfindungsgemäße Aufgabenstellung insbesondere dann vollständig und in zufrieden stellender Weise gelöst werden kann, wenn die erfindungsgemäßen Mittel weitere ausgewählte Formulierungsbestandteile enthalten.

So hat sich herausgestellt, dass die zusätzliche Anwesenheit von bestimmten, höherkettigen Fettalkoholen das geruchliche Ergebnis der erfindungsgemäßen Zusammensetzungen noch weiter verbessert. Erfindungsgemäß bevorzugte Mittel sind daher dadurch gekennzeichnet, dass zusätzlich mindestens ein lineares gesättigtes Alkanol mit 12-18 Kohlenstoffatomen, insbesondere Cetylalkohol, Stearylalkohol oder Lanolinalkohol oder Gemische dieser Alkohole, wie sie bei der großtechnischen Hydrierung von pflanzlichen und tierischen Fettsäuren erhältlich sind, sowie Mischungen dieser Alkanole, insbesondere Cetylalkohol oder Stearylalkohol oder Mischungen hiervon, enthalten sind, bevorzugt in einer Gesamtmenge von 0,1 bis 10 Gew.-%, besonders bevorzugt 1 bis 7 Gew.-%, außerordentlich bevorzugt 2 bis 5 Gew.-%, jeweils bezogen auf das Gewicht des Mittels.

Weiterhin hat sich herausgestellt, dass die zusätzliche Anwesenheit von bestimmten ethoxylierten Fettalkoholen mit einem Ethoxylierungsgrad von 80 bis 120 das geruchliche Ergebnis der erfindungsgemäßen Zusammensetzungen noch weiter verbessert.

In einer weiteren ganz besonders bevorzugten Ausführungsform ist ein Mittel zum Glätten, permanenten Umformen oder zur Depilation von keratinischen Fasern dadurch gekennzeichnet, dass es als ethoxylierte(n) Fettalkohol(e) mit einem Ethoxylierungsgrad von 80 bis 120 eine oder mehrere Verbindungen der Formel (III) enthält, 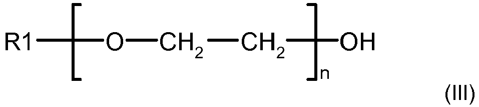
worin R1 für eine gesättigte oder ungesättigte, unverzweigte oder verzweigte C₈-C₂₄-Alkylgruppe, bevorzugt für eine gesättigte, unverzeigte C₁₆- bis C₁₈- Alkylgruppe, steht und
n für eine ganze Zahl von 80 bis 120, bevorzugt für eine ganze Zahl von 90 bis 110 und besonders bevorzugt für die Zahl 100, steht.

Unter Fettalkoholen sind erfindungsgemäß gesättigte oder ungesättigte, unverzweigte oder verzweigte C₈-C₂₄-Alkylgruppen mit Hydroxysubstitution zu verstehen. Ungesättigte Fettalkohole können einfach oder mehrfach ungesättigt sein. Bei einem ungesättigten Fettalkohol kann bzw. können deren C-C-Doppelbindung(en) die Cis- oder Trans-Konfiguration aufweisen.

Bevorzugte Fettalkohole sind Octan-1-ol (Octylalkohol, Caprylalkohol), Decan-1-ol (Decylalkohol, Caprinalkohol), Dodecan-1-ol (Dodecylalkohol, Laurylalkohol), Tetradecan-1-ol, (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), (9*Z*)-Octadec-9-en-1-ol (Oleylalkohol), (9*E*)-Octadec-9-en-1-ol (Elaidylalkohol), (9*Z*,12*Z*)-Octadeca-9,12-dien-1-ol (Linoleylalkohol), (9*Z*,12*Z*,15*Z*)-Octadeca-9,12,15-trien-1-ol (Linolenoylalkohol), Eicosan-1-ol (Eicosylalkohol, Arachylalkohol), (9*Z*)-Eicos-9-en-1-ol (Gadoleylalkohol), (5*Z*,8*Z*,11*Z*,14*Z*)-Eicosa-5,8,11,14-tetraen-1-ol (Arachidonalkohol), Docosan-1-ol (Docosylalkohol, Behenylalkohol), (13*E*)-Docosen-1-ol (Brassidylalkohol) und (13*Z*)-Docos-13-en-1-ol (Erucylalkohol). Innerhalb dieser Gruppe wiederum sind Hexadecan-1-ol (Hexadecylalkohol, Cetylalkohol, Palmitylalkohol) und Octadecan-1-ol (Octadecylalkohol, Stearylalkohol) ganz besonders bevorzugte Fettalkohole.

Zur Ausbildung des optionalen Bestandteils sind diese Fettalkohole mit einem Ethoxylierungsgrad von 80 bis 120 ethoxyliert.

Unter Ethoxylierung (auch Oxyethylierung) wird die Reaktion der Fettalkohole mit Ethylenoxid (EO) verstanden. Durch Insertion von 80 bis 120 Gruppierungen des Typs -CH2-CH2-O- pro Fettalkohol Molekül entstehen lineare Polyether, die an einem Kettenende eine Hydroxygruppe und am anderen Kettenende die C₈-C₂₄-Alkylgruppe des Fettalkohols tragen.

Bevorzugte ethoxylierte Fettalkohole besitzen einen Ethoxylierungsgrad von 90 bis 110. Ganz besonders bevorzugt ist es, wenn ethoxylierte Fettalkohole eingesetzt werden, die einen Ethoxylierungsgrad von 100 besitzen.

Im Zuge der zu dieser Erfindung führenden Arbeiten hat sich gezeigt, dass der Ethoxylierungsgrad des ethoxylierten Fettalkohols überraschenderweise die Fähigkeit des Mittels zur Reduzierung des schwefligen Geruchs weiter verbessern kann. Aus diesem Grund ist es besonders bevorzugt, wenn als ethoxylierte(r) Fettalkohol(e) ein oder mehrere ethoxylierte Fettalkohole mit einem ganz bestimmten Ethoxylierungsgrad eingesetzt werden. Ganz besonders bevorzugt ist es, wenn als ethoxylierter Fettalkohol ein oder mehrere ethoxylierte Fettalkohole aus der Gruppe
- Dodecan-1-ol (Dodecylalkohol, Laurylalkohol), ethoxyliert mit 80 EO,
- Dodecan-1-ol (Dodecylalkohol, Laurylalkohol), ethoxyliert mit 81 EO,
- Dodecan-1-ol (Dodecylalkohol, Laurylalkohol), ethoxyliert mit 82 EO,
- Dodecan-1-ol (Dodecylalkohol, Laurylalkohol), ethoxyliert mit 83 EO,
- Dodecan-1-ol (Dodecylalkohol, Laurylalkohol), ethoxyliert mit 84 EO,
- Dodecan-1-ol (Dodecylalkohol, Laurylalkohol), ethoxyliert mit 85 EO,
- Dodecan-1-ol (Dodecylalkohol, Laurylalkohol), ethoxyliert mit 86 EO,
- Dodecan-1-ol (Dodecylalkohol, Laurylalkohol), ethoxyliert mit 87 EO,
- Dodecan-1-ol (Dodecylalkohol, Laurylalkohol), ethoxyliert mit 88 EO,
- Dodecan-1-ol (Dodecylalkohol, Laurylalkohol), ethoxyliert mit 89 EO,
- Dodecan-1-ol (Dodecylalkohol, Laurylalkohol), ethoxyliert mit 90 EO,
- Dodecan-1-ol (Dodecylalkohol, Laurylalkohol), ethoxyliert mit 91 EO,
- Dodecan-1-ol (Dodecylalkohol, Laurylalkohol), ethoxyliert mit 92 EO,
- Dodecan-1-ol (Dodecylalkohol, Laurylalkohol), ethoxyliert mit 93 EO,
- Dodecan-1-ol (Dodecylalkohol, Laurylalkohol), ethoxyliert mit 94 EO,
- Dodecan-1-ol (Dodecylalkohol, Laurylalkohol), ethoxyliert mit 95 EO,
- Dodecan-1-ol (Dodecylalkohol, Laurylalkohol), ethoxyliert mit 96 EO,
- Dodecan-1-ol (Dodecylalkohol, Laurylalkohol), ethoxyliert mit 97 EO,
- Dodecan-1-ol (Dodecylalkohol, Laurylalkohol), ethoxyliert mit 98 EO,
- Dodecan-1-ol (Dodecylalkohol, Laurylalkohol), ethoxyliert mit 99 EO,
- Dodecan-1-ol (Dodecylalkohol, Laurylalkohol), ethoxyliert mit 100 EO,
- Dodecan-1-ol (Dodecylalkohol, Laurylalkohol), ethoxyliert mit 101 EO,
- Dodecan-1-ol (Dodecylalkohol, Laurylalkohol), ethoxyliert mit 102 EO,
- Dodecan-1-ol (Dodecylalkohol, Laurylalkohol), ethoxyliert mit 103 EO,
- Dodecan-1-ol (Dodecylalkohol, Laurylalkohol), ethoxyliert mit 104 EO,
- Dodecan-1-ol (Dodecylalkohol, Laurylalkohol), ethoxyliert mit 105 EO,
- Dodecan-1-ol (Dodecylalkohol, Laurylalkohol), ethoxyliert mit 106 EO,
- Dodecan-1-ol (Dodecylalkohol, Laurylalkohol), ethoxyliert mit 107 EO,
- Dodecan-1-ol (Dodecylalkohol, Laurylalkohol), ethoxyliert mit 108 EO,
- Dodecan-1-ol (Dodecylalkohol, Laurylalkohol), ethoxyliert mit 109 EO,
- Dodecan-1-ol (Dodecylalkohol, Laurylalkohol), ethoxyliert mit 110 EO,
- Dodecan-1-ol (Dodecylalkohol, Laurylalkohol), ethoxyliert mit 111 EO,
- Dodecan-1-ol (Dodecylalkohol, Laurylalkohol), ethoxyliert mit 112 EO,
- Dodecan-1-ol (Dodecylalkohol, Laurylalkohol), ethoxyliert mit 113 EO,
- Dodecan-1-ol (Dodecylalkohol, Laurylalkohol), ethoxyliert mit 114 EO,
- Dodecan-1-ol (Dodecylalkohol, Laurylalkohol), ethoxyliert mit 115 EO,
- Dodecan-1-ol (Dodecylalkohol, Laurylalkohol), ethoxyliert mit 116 EO,
- Dodecan-1-ol (Dodecylalkohol, Laurylalkohol), ethoxyliert mit 117 EO,
- Dodecan-1-ol (Dodecylalkohol, Laurylalkohol), ethoxyliert mit 118 EO,
- Dodecan-1-ol (Dodecylalkohol, Laurylalkohol), ethoxyliert mit 119 EO,
- Dodecan-1-ol (Dodecylalkohol, Laurylalkohol), ethoxyliert mit 120 EO,
- Tetradecan-1-ol, (Tetradecylalkohol, Myristylalkohol), ethoxyliert mit 80 EO,
- Tetradecan-1-ol, (Tetradecylalkohol, Myristylalkohol), ethoxyliert mit 81 EO,
- Tetradecan-1-ol, (Tetradecylalkohol, Myristylalkohol), ethoxyliert mit 82 EO,
- Tetradecan-1-ol, (Tetradecylalkohol, Myristylalkohol), ethoxyliert mit 83 EO,
- Tetradecan-1-ol, (Tetradecylalkohol, Myristylalkohol), ethoxyliert mit 84 EO,
- Tetradecan-1-ol, (Tetradecylalkohol, Myristylalkohol), ethoxyliert mit 85 EO,
- Tetradecan-1-ol, (Tetradecylalkohol, Myristylalkohol), ethoxyliert mit 86 EO,
- Tetradecan-1-ol, (Tetradecylalkohol, Myristylalkohol), ethoxyliert mit 87 EO,
- Tetradecan-1-ol, (Tetradecylalkohol, Myristylalkohol), ethoxyliert mit 88 EO,
- Tetradecan-1-ol, (Tetradecylalkohol, Myristylalkohol), ethoxyliert mit 89 EO,
- Tetradecan-1-ol, (Tetradecylalkohol, Myristylalkohol), ethoxyliert mit 90 EO,
- Tetradecan-1-ol, (Tetradecylalkohol, Myristylalkohol), ethoxyliert mit 91 EO,
- Tetradecan-1-ol, (Tetradecylalkohol, Myristylalkohol), ethoxyliert mit 92 EO,
- Tetradecan-1-ol, (Tetradecylalkohol, Myristylalkohol), ethoxyliert mit 93 EO,
- Tetradecan-1-ol, (Tetradecylalkohol, Myristylalkohol), ethoxyliert mit 94 EO,
- Tetradecan-1-ol, (Tetradecylalkohol, Myristylalkohol), ethoxyliert mit 95 EO,
- Tetradecan-1-ol, (Tetradecylalkohol, Myristylalkohol), ethoxyliert mit 96 EO,
- Tetradecan-1-ol, (Tetradecylalkohol, Myristylalkohol), ethoxyliert mit 97 EO,
- Tetradecan-1-ol, (Tetradecylalkohol, Myristylalkohol), ethoxyliert mit 98 EO,
- Tetradecan-1-ol, (Tetradecylalkohol, Myristylalkohol), ethoxyliert mit 99 EO,
- Tetradecan-1-ol, (Tetradecylalkohol, Myristylalkohol), ethoxyliert mit 100 EO,
- Tetradecan-1-ol, (Tetradecylalkohol, Myristylalkohol), ethoxyliert mit 101 EO,
- Tetradecan-1-ol, (Tetradecylalkohol, Myristylalkohol), ethoxyliert mit 102 EO,
- Tetradecan-1-ol, (Tetradecylalkohol, Myristylalkohol), ethoxyliert mit 103 EO,
- Tetradecan-1-ol, (Tetradecylalkohol, Myristylalkohol), ethoxyliert mit 104 EO,
- Tetradecan-1-ol, (Tetradecylalkohol, Myristylalkohol), ethoxyliert mit 105 EO,
- Tetradecan-1-ol, (Tetradecylalkohol, Myristylalkohol), ethoxyliert mit 106 EO,
- Tetradecan-1-ol, (Tetradecylalkohol, Myristylalkohol), ethoxyliert mit 107 EO,
- Tetradecan-1-ol, (Tetradecylalkohol, Myristylalkohol), ethoxyliert mit 108 EO,
- Tetradecan-1-ol, (Tetradecylalkohol, Myristylalkohol), ethoxyliert mit 109 EO,
- Tetradecan-1-ol, (Tetradecylalkohol, Myristylalkohol), ethoxyliert mit 110 EO,
- Tetradecan-1-ol, (Tetradecylalkohol, Myristylalkohol), ethoxyliert mit 111 EO,
- Tetradecan-1-ol, (Tetradecylalkohol, Myristylalkohol), ethoxyliert mit 112 EO,
- Tetradecan-1-ol, (Tetradecylalkohol, Myristylalkohol), ethoxyliert mit 113 EO,
- Tetradecan-1-ol, (Tetradecylalkohol, Myristylalkohol), ethoxyliert mit 114 EO,
- Tetradecan-1-ol, (Tetradecylalkohol, Myristylalkohol), ethoxyliert mit 115 EO,
- Tetradecan-1-ol, (Tetradecylalkohol, Myristylalkohol), ethoxyliert mit 116 EO,
- Tetradecan-1-ol, (Tetradecylalkohol, Myristylalkohol), ethoxyliert mit 117 EO,
- Tetradecan-1-ol, (Tetradecylalkohol, Myristylalkohol), ethoxyliert mit 118 EO,
- Tetradecan-1-ol, (Tetradecylalkohol, Myristylalkohol), ethoxyliert mit 119 EO,
- Tetradecan-1-ol, (Tetradecylalkohol, Myristylalkohol), ethoxyliert mit 120 EO,
- Hexadecan-1-ol (Hexadecylalkohol, Cetylalkohol, Palmitylalkohol), ethoxyliert mit 80 EO,
- Hexadecan-1-ol (Hexadecylalkohol, Cetylalkohol, Palmitylalkohol), ethoxyliert mit 81 EO,
- Hexadecan-1-ol (Hexadecylalkohol, Cetylalkohol, Palmitylalkohol), ethoxyliert mit 82 EO,
- Hexadecan-1-ol (Hexadecylalkohol, Cetylalkohol, Palmitylalkohol), ethoxyliert mit 83 EO,
- Hexadecan-1-ol (Hexadecylalkohol, Cetylalkohol, Palmitylalkohol), ethoxyliert mit 84 EO,
- Hexadecan-1-ol (Hexadecylalkohol, Cetylalkohol, Palmitylalkohol), ethoxyliert mit 85 EO,
- Hexadecan-1-ol (Hexadecylalkohol, Cetylalkohol, Palmitylalkohol), ethoxyliert mit 86 EO,
- Hexadecan-1-ol (Hexadecylalkohol, Cetylalkohol, Palmitylalkohol), ethoxyliert mit 87 EO,
- Hexadecan-1-ol (Hexadecylalkohol, Cetylalkohol, Palmitylalkohol), ethoxyliert mit 88 EO,
- Hexadecan-1-ol (Hexadecylalkohol, Cetylalkohol, Palmitylalkohol), ethoxyliert mit 89 EO,
- Hexadecan-1-ol (Hexadecylalkohol, Cetylalkohol, Palmitylalkohol), ethoxyliert mit 90 EO,
- Hexadecan-1-ol (Hexadecylalkohol, Cetylalkohol, Palmitylalkohol), ethoxyliert mit 91 EO,
- Hexadecan-1-ol (Hexadecylalkohol, Cetylalkohol, Palmitylalkohol), ethoxyliert mit 92 EO,
- Hexadecan-1-ol (Hexadecylalkohol, Cetylalkohol, Palmitylalkohol), ethoxyliert mit 93 EO,
- Hexadecan-1-ol (Hexadecylalkohol, Cetylalkohol, Palmitylalkohol), ethoxyliert mit 94 EO,
- Hexadecan-1-ol (Hexadecylalkohol, Cetylalkohol, Palmitylalkohol), ethoxyliert mit 95 EO,
- Hexadecan-1-ol (Hexadecylalkohol, Cetylalkohol, Palmitylalkohol), ethoxyliert mit 96 EO,
- Hexadecan-1-ol (Hexadecylalkohol, Cetylalkohol, Palmitylalkohol), ethoxyliert mit 97 EO,
- Hexadecan-1-ol (Hexadecylalkohol, Cetylalkohol, Palmitylalkohol), ethoxyliert mit 98 EO,
- Hexadecan-1-ol (Hexadecylalkohol, Cetylalkohol, Palmitylalkohol), ethoxyliert mit 99 EO,
- Hexadecan-1-ol (Hexadecylalkohol, Cetylalkohol, Palmitylalkohol), ethoxyliert mit 100 EO,
- Hexadecan-1-ol (Hexadecylalkohol, Cetylalkohol, Palmitylalkohol), ethoxyliert mit 101 EO,
- Hexadecan-1-ol (Hexadecylalkohol, Cetylalkohol, Palmitylalkohol), ethoxyliert mit 102 EO,
- Hexadecan-1-ol (Hexadecylalkohol, Cetylalkohol, Palmitylalkohol), ethoxyliert mit 103 EO,
- Hexadecan-1-ol (Hexadecylalkohol, Cetylalkohol, Palmitylalkohol), ethoxyliert mit 104 EO,
- Hexadecan-1-ol (Hexadecylalkohol, Cetylalkohol, Palmitylalkohol), ethoxyliert mit 105 EO,
- Hexadecan-1-ol (Hexadecylalkohol, Cetylalkohol, Palmitylalkohol), ethoxyliert mit 106 EO,
- Hexadecan-1-ol (Hexadecylalkohol, Cetylalkohol, Palmitylalkohol), ethoxyliert mit 107 EO,
- Hexadecan-1-ol (Hexadecylalkohol, Cetylalkohol, Palmitylalkohol), ethoxyliert mit 108 EO,
- Hexadecan-1-ol (Hexadecylalkohol, Cetylalkohol, Palmitylalkohol), ethoxyliert mit 109 EO,
- Hexadecan-1-ol (Hexadecylalkohol, Cetylalkohol, Palmitylalkohol), ethoxyliert mit 110 EO,
- Hexadecan-1-ol (Hexadecylalkohol, Cetylalkohol, Palmitylalkohol), ethoxyliert mit 111 EO,
- Hexadecan-1-ol (Hexadecylalkohol, Cetylalkohol, Palmitylalkohol), ethoxyliert mit 112 EO,
- Hexadecan-1-ol (Hexadecylalkohol, Cetylalkohol, Palmitylalkohol), ethoxyliert mit 113 EO,
- Hexadecan-1-ol (Hexadecylalkohol, Cetylalkohol, Palmitylalkohol), ethoxyliert mit 114 EO,
- Hexadecan-1-ol (Hexadecylalkohol, Cetylalkohol, Palmitylalkohol), ethoxyliert mit 115 EO,
- Hexadecan-1-ol (Hexadecylalkohol, Cetylalkohol, Palmitylalkohol), ethoxyliert mit 116 EO,
- Hexadecan-1-ol (Hexadecylalkohol, Cetylalkohol, Palmitylalkohol), ethoxyliert mit 117 EO,
- Hexadecan-1-ol (Hexadecylalkohol, Cetylalkohol, Palmitylalkohol), ethoxyliert mit 118 EO,
- Hexadecan-1-ol (Hexadecylalkohol, Cetylalkohol, Palmitylalkohol), ethoxyliert mit 119 EO,
- Hexadecan-1-ol (Hexadecylalkohol, Cetylalkohol, Palmitylalkohol), ethoxyliert mit 120 EO,
- Octadecan-1-ol (Octadecylalkohol, Stearylalkohol), ethoxyliert mit 80 EO,
- Octadecan-1-ol (Octadecylalkohol, Stearylalkohol), ethoxyliert mit 81 EO,
- Octadecan-1-ol (Octadecylalkohol, Stearylalkohol), ethoxyliert mit 82 EO,
- Octadecan-1-ol (Octadecylalkohol, Stearylalkohol), ethoxyliert mit 83 EO,
- Octadecan-1-ol (Octadecylalkohol, Stearylalkohol), ethoxyliert mit 84 EO,
- Octadecan-1-ol (Octadecylalkohol, Stearylalkohol), ethoxyliert mit 85 EO,
- Octadecan-1-ol (Octadecylalkohol, Stearylalkohol), ethoxyliert mit 86 EO,
- Octadecan-1-ol (Octadecylalkohol, Stearylalkohol), ethoxyliert mit 87 EO,
- Octadecan-1-ol (Octadecylalkohol, Stearylalkohol), ethoxyliert mit 88 EO,
- Octadecan-1-ol (Octadecylalkohol, Stearylalkohol), ethoxyliert mit 89 EO,
- Octadecan-1-ol (Octadecylalkohol, Stearylalkohol), ethoxyliert mit 90 EO,
- Octadecan-1-ol (Octadecylalkohol, Stearylalkohol), ethoxyliert mit 91 EO,
- Octadecan-1-ol (Octadecylalkohol, Stearylalkohol), ethoxyliert mit 92 EO,
- Octadecan-1-ol (Octadecylalkohol, Stearylalkohol), ethoxyliert mit 93 EO,
- Octadecan-1-ol (Octadecylalkohol, Stearylalkohol), ethoxyliert mit 94 EO,
- Octadecan-1-ol (Octadecylalkohol, Stearylalkohol), ethoxyliert mit 95 EO,
- Octadecan-1-ol (Octadecylalkohol, Stearylalkohol), ethoxyliert mit 96 EO,
- Octadecan-1-ol (Octadecylalkohol, Stearylalkohol), ethoxyliert mit 97 EO,
- Octadecan-1-ol (Octadecylalkohol, Stearylalkohol), ethoxyliert mit 98 EO,
- Octadecan-1-ol (Octadecylalkohol, Stearylalkohol), ethoxyliert mit 99 EO,
- Octadecan-1-ol (Octadecylalkohol, Stearylalkohol), ethoxyliert mit 100 EO,
- Octadecan-1-ol (Octadecylalkohol, Stearylalkohol), ethoxyliert mit 101 EO,
- Octadecan-1-ol (Octadecylalkohol, Stearylalkohol), ethoxyliert mit 102 EO,
- Octadecan-1-ol (Octadecylalkohol, Stearylalkohol), ethoxyliert mit 103 EO,
- Octadecan-1-ol (Octadecylalkohol, Stearylalkohol), ethoxyliert mit 104 EO,
- Octadecan-1-ol (Octadecylalkohol, Stearylalkohol), ethoxyliert mit 105 EO,
- Octadecan-1-ol (Octadecylalkohol, Stearylalkohol), ethoxyliert mit 106 EO,
- Octadecan-1-ol (Octadecylalkohol, Stearylalkohol), ethoxyliert mit 107 EO,
- Octadecan-1-ol (Octadecylalkohol, Stearylalkohol), ethoxyliert mit 108 EO,
- Octadecan-1-ol (Octadecylalkohol, Stearylalkohol), ethoxyliert mit 109 EO,
- Octadecan-1-ol (Octadecylalkohol, Stearylalkohol), ethoxyliert mit 110 EO,
- Octadecan-1-ol (Octadecylalkohol, Stearylalkohol), ethoxyliert mit 111 EO,
- Octadecan-1-ol (Octadecylalkohol, Stearylalkohol), ethoxyliert mit 112 EO,
- Octadecan-1-ol (Octadecylalkohol, Stearylalkohol), ethoxyliert mit 113 EO,
- Octadecan-1-ol (Octadecylalkohol, Stearylalkohol), ethoxyliert mit 114 EO,
- Octadecan-1-ol (Octadecylalkohol, Stearylalkohol), ethoxyliert mit 115 EO,
- Octadecan-1-ol (Octadecylalkohol, Stearylalkohol), ethoxyliert mit 116 EO,
- Octadecan-1-ol (Octadecylalkohol, Stearylalkohol), ethoxyliert mit 117 EO,
- Octadecan-1-ol (Octadecylalkohol, Stearylalkohol), ethoxyliert mit 118 EO,
- Octadecan-1-ol (Octadecylalkohol, Stearylalkohol), ethoxyliert mit 119 EO,
- Octadecan-1-ol (Octadecylalkohol, Stearylalkohol), ethoxyliert mit 120 EO,
- (9*Z*)-Octadec-9-en-1-ol (Oleylalkohol), ethoxyliert mit 80 EO,
- (9*Z*)-Octadec-9-en-1-ol (Oleylalkohol), ethoxyliert mit 81 EO,
- (9*Z*)-Octadec-9-en-1-ol (Oleylalkohol), ethoxyliert mit 82 EO,
- (9*Z*)-Octadec-9-en-1-ol (Oleylalkohol), ethoxyliert mit 83 EO,
- (9*Z*)-Octadec-9-en-1-ol (Oleylalkohol), ethoxyliert mit 84 EO,
- (9*Z*)-Octadec-9-en-1-ol (Oleylalkohol), ethoxyliert mit 85 EO,
- (9*Z*)-Octadec-9-en-1-ol (Oleylalkohol), ethoxyliert mit 86 EO,
- (9*Z*)-Octadec-9-en-1-ol (Oleylalkohol), ethoxyliert mit 87 EO,
- (9*Z*)-Octadec-9-en-1-ol (Oleylalkohol), ethoxyliert mit 88 EO,
- (9*Z*)-Octadec-9-en-1-ol (Oleylalkohol), ethoxyliert mit 89 EO,
- (9*Z*)-Octadec-9-en-1-ol (Oleylalkohol), ethoxyliert mit 90 EO,
- (9*Z*)-Octadec-9-en-1-ol (Oleylalkohol), ethoxyliert mit 91 EO,
- (9*Z*)-Octadec-9-en-1-ol (Oleylalkohol), ethoxyliert mit 92 EO,
- (9*Z*)-Octadec-9-en-1-ol (Oleylalkohol), ethoxyliert mit 93 EO,
- (9*Z*)-Octadec-9-en-1-ol (Oleylalkohol), ethoxyliert mit 94 EO,
- (9*Z*)-Octadec-9-en-1-ol (Oleylalkohol), ethoxyliert mit 95 EO,
- (9*Z*)-Octadec-9-en-1-ol (Oleylalkohol), ethoxyliert mit 96 EO,
- (9*Z*)-Octadec-9-en-1-ol (Oleylalkohol), ethoxyliert mit 97 EO,
- (9*Z*)-Octadec-9-en-1-ol (Oleylalkohol), ethoxyliert mit 98 EO,
- (9*Z*)-Octadec-9-en-1-ol (Oleylalkohol), ethoxyliert mit 99 EO,
- (9*Z*)-Octadec-9-en-1-ol (Oleylalkohol), ethoxyliert mit 100 EO,
- (9*Z*)-Octadec-9-en-1-ol (Oleylalkohol), ethoxyliert mit 101 EO,
- (9*Z*)-Octadec-9-en-1-ol (Oleylalkohol), ethoxyliert mit 102 EO,
- (9*Z*)-Octadec-9-en-1-ol (Oleylalkohol), ethoxyliert mit 103 EO,
- (9*Z*)-Octadec-9-en-1-ol (Oleylalkohol), ethoxyliert mit 104 EO,
- (9*Z*)-Octadec-9-en-1-ol (Oleylalkohol), ethoxyliert mit 105 EO,
- (9*Z*)-Octadec-9-en-1-ol (Oleylalkohol), ethoxyliert mit 106 EO,
- (9*Z*)-Octadec-9-en-1-ol (Oleylalkohol), ethoxyliert mit 107 EO,
- (9*Z*)-Octadec-9-en-1-ol (Oleylalkohol), ethoxyliert mit 108 EO,
- (9*Z*)-Octadec-9-en-1-ol (Oleylalkohol), ethoxyliert mit 109 EO,
- (9*Z*)-Octadec-9-en-1-ol (Oleylalkohol), ethoxyliert mit 110 EO,
- (9*Z*)-Octadec-9-en-1-ol (Oleylalkohol), ethoxyliert mit 111 EO,
- (9*Z*)-Octadec-9-en-1-ol (Oleylalkohol), ethoxyliert mit 112 EO,
- (9*Z*)-Octadec-9-en-1-ol (Oleylalkohol), ethoxyliert mit 113 EO,
- (9*Z*)-Octadec-9-en-1-ol (Oleylalkohol), ethoxyliert mit 114 EO,
- (9*Z*)-Octadec-9-en-1-ol (Oleylalkohol), ethoxyliert mit 115 EO,
- (9*Z*)-Octadec-9-en-1-ol (Oleylalkohol), ethoxyliert mit 116 EO,
- (9*Z*)-Octadec-9-en-1-ol (Oleylalkohol), ethoxyliert mit 117 EO,
- (9*Z*)-Octadec-9-en-1-ol (Oleylalkohol), ethoxyliert mit 118 EO,
- (9*Z*)-Octadec-9-en-1-ol (Oleylalkohol), ethoxyliert mit 119 EO,
- (9*Z*)-Octadec-9-en-1-ol (Oleylalkohol), ethoxyliert mit 120 EO,
- (9*E*)-Octadec-9-en-1-ol (Elaidylalkohol), ethoxyliert mit 80 EO,
- (9*E*)-Octadec-9-en-1-ol (Elaidylalkohol), ethoxyliert mit 81 EO,
- (9*E*)-Octadec-9-en-1-ol (Elaidylalkohol), ethoxyliert mit 82 EO,
- (9*E*)-Octadec-9-en-1-ol (Elaidylalkohol), ethoxyliert mit 83 EO,
- (9*E*)-Octadec-9-en-1-ol (Elaidylalkohol), ethoxyliert mit 84 EO,
- (9*E*)-Octadec-9-en-1-ol (Elaidylalkohol), ethoxyliert mit 85 EO,
- (9*E*)-Octadec-9-en-1-ol (Elaidylalkohol), ethoxyliert mit 86 EO,
- (9*E*)-Octadec-9-en-1-ol (Elaidylalkohol), ethoxyliert mit 87 EO,
- (9*E*)-Octadec-9-en-1-ol (Elaidylalkohol), ethoxyliert mit 88 EO,
- (9*E*)-Octadec-9-en-1-ol (Elaidylalkohol), ethoxyliert mit 89 EO,
- (9*E*)-Octadec-9-en-1-ol (Elaidylalkohol), ethoxyliert mit 90 EO,
- (9*E*)-Octadec-9-en-1-ol (Elaidylalkohol), ethoxyliert mit 91 EO,
- (9*E*)-Octadec-9-en-1-ol (Elaidylalkohol), ethoxyliert mit 92 EO,
- (9*E*)-Octadec-9-en-1-ol (Elaidylalkohol), ethoxyliert mit 93 EO,
- (9*E*)-Octadec-9-en-1-ol (Elaidylalkohol), ethoxyliert mit 94 EO,
- (9*E*)-Octadec-9-en-1-ol (Elaidylalkohol), ethoxyliert mit 95 EO,
- (9*E*)-Octadec-9-en-1-ol (Elaidylalkohol), ethoxyliert mit 96 EO,
- (9*E*)-Octadec-9-en-1-ol (Elaidylalkohol), ethoxyliert mit 97 EO,
- (9*E*)-Octadec-9-en-1-ol (Elaidylalkohol), ethoxyliert mit 98 EO,
- (9*E*)-Octadec-9-en-1-ol (Elaidylalkohol), ethoxyliert mit 99 EO,
- (9*E*)-Octadec-9-en-1-ol (Elaidylalkohol), ethoxyliert mit 100 EO,
- (9*E*)-Octadec-9-en-1-ol (Elaidylalkohol), ethoxyliert mit 101 EO,
- (9*E*)-Octadec-9-en-1-ol (Elaidylalkohol), ethoxyliert mit 102 EO,
- (9*E*)-Octadec-9-en-1-ol (Elaidylalkohol), ethoxyliert mit 103 EO,
- (9*E*)-Octadec-9-en-1-ol (Elaidylalkohol), ethoxyliert mit 104 EO,
- (9*E*)-Octadec-9-en-1-ol (Elaidylalkohol), ethoxyliert mit 105 EO,
- (9*E*)-Octadec-9-en-1-ol (Elaidylalkohol), ethoxyliert mit 106 EO,
- (9*E*)-Octadec-9-en-1-ol (Elaidylalkohol), ethoxyliert mit 107 EO,
- (9*E*)-Octadec-9-en-1-ol (Elaidylalkohol), ethoxyliert mit 108 EO,
- (9*E*)-Octadec-9-en-1-ol (Elaidylalkohol), ethoxyliert mit 109 EO,
- (9*E*)-Octadec-9-en-1-ol (Elaidylalkohol), ethoxyliert mit 110 EO,
- (9*E*)-Octadec-9-en-1-ol (Elaidylalkohol), ethoxyliert mit 111 EO,
- (9*E*)-Octadec-9-en-1-ol (Elaidylalkohol), ethoxyliert mit 112 EO,
- (9*E*)-Octadec-9-en-1-ol (Elaidylalkohol), ethoxyliert mit 113 EO,
- (9*E*)-Octadec-9-en-1-ol (Elaidylalkohol), ethoxyliert mit 114 EO,
- (9*E*)-Octadec-9-en-1-ol (Elaidylalkohol), ethoxyliert mit 115 EO,
- (9*E*)-Octadec-9-en-1-ol (Elaidylalkohol), ethoxyliert mit 116 EO,
- (9*E*)-Octadec-9-en-1-ol (Elaidylalkohol), ethoxyliert mit 117 EO,
- (9*E*)-Octadec-9-en-1-ol (Elaidylalkohol), ethoxyliert mit 118 EO,
- (9*E*)-Octadec-9-en-1-ol (Elaidylalkohol), ethoxyliert mit 119 EO,
- (9*E*)-Octadec-9-en-1-ol (Elaidylalkohol), ethoxyliert mit 120 EO,
- (9*Z*,12*Z*)-Octadeca-9,12-dien-1-ol (Linoleylalkohol), ethoxyliert mit 80 EO,
- (9*Z*,12*Z*)-Octadeca-9,12-dien-1-ol (Linoleylalkohol), ethoxyliert mit 81 EO,
- (9*Z*,12*Z*)-Octadeca-9,12-dien-1-ol (Linoleylalkohol), ethoxyliert mit 82 EO,
- (9*Z*,12*Z*)-Octadeca-9,12-dien-1-ol (Linoleylalkohol), ethoxyliert mit 83 EO,
- (9*Z*,12*Z*)-Octadeca-9,12-dien-1-ol (Linoleylalkohol), ethoxyliert mit 84 EO,
- (9*Z*,12*Z*)-Octadeca-9,12-dien-1-ol (Linoleylalkohol), ethoxyliert mit 85 EO,
- (9*Z*,12*Z*)-Octadeca-9,12-dien-1-ol (Linoleylalkohol), ethoxyliert mit 86 EO,
- (9*Z*,12*Z*)-Octadeca-9,12-dien-1-ol (Linoleylalkohol), ethoxyliert mit 87 EO,
- (9*Z*,12*Z*)-Octadeca-9,12-dien-1-ol (Linoleylalkohol), ethoxyliert mit 88 EO,
- (9*Z*,12*Z*)-Octadeca-9,12-dien-1-ol (Linoleylalkohol), ethoxyliert mit 89 EO,
- (9*Z*,12*Z*)-Octadeca-9,12-dien-1-ol (Linoleylalkohol), ethoxyliert mit 90 EO,
- (9*Z*,12*Z*)-Octadeca-9,12-dien-1-ol (Linoleylalkohol), ethoxyliert mit 91 EO,
- (9*Z*,12*Z*)-Octadeca-9,12-dien-1-ol (Linoleylalkohol), ethoxyliert mit 92 EO,
- (9*Z*,12*Z*)-Octadeca-9,12-dien-1-ol (Linoleylalkohol), ethoxyliert mit 93 EO,
- (9*Z*,12*Z*)-Octadeca-9,12-dien-1-ol (Linoleylalkohol), ethoxyliert mit 94 EO,
- (9*Z*,12*Z*)-Octadeca-9,12-dien-1-ol (Linoleylalkohol), ethoxyliert mit 95 EO,
- (9*Z*,12*Z*)-Octadeca-9,12-dien-1-ol (Linoleylalkohol), ethoxyliert mit 96 EO,
- (9*Z*,12*Z*)-Octadeca-9,12-dien-1-ol (Linoleylalkohol), ethoxyliert mit 97 EO,
- (9*Z*,12*Z*)-Octadeca-9,12-dien-1-ol (Linoleylalkohol), ethoxyliert mit 98 EO,
- (9*Z*,12*Z*)-Octadeca-9,12-dien-1-ol (Linoleylalkohol), ethoxyliert mit 99 EO,
- (9*Z*,12*Z*)-Octadeca-9,12-dien-1-ol (Linoleylalkohol), ethoxyliert mit 100 EO,
- (9*Z*,12*Z*)-Octadeca-9,12-dien-1-ol (Linoleylalkohol), ethoxyliert mit 101 EO,
- (9*Z*,12*Z*)-Octadeca-9,12-dien-1-ol (Linoleylalkohol), ethoxyliert mit 102 EO,
- (9*Z*,12*Z*)-Octadeca-9,12-dien-1-ol (Linoleylalkohol), ethoxyliert mit 103 EO,
- (9*Z*,12*Z*)-Octadeca-9,12-dien-1-ol (Linoleylalkohol), ethoxyliert mit 104 EO,
- (9*Z*,12*Z*)-Octadeca-9,12-dien-1-ol (Linoleylalkohol), ethoxyliert mit 105 EO,
- (9*Z*,12*Z*)-Octadeca-9,12-dien-1-ol (Linoleylalkohol), ethoxyliert mit 106 EO,
- (9*Z*,12*Z*)-Octadeca-9,12-dien-1-ol (Linoleylalkohol), ethoxyliert mit 107 EO,
- (9*Z*,12*Z*)-Octadeca-9,12-dien-1-ol (Linoleylalkohol), ethoxyliert mit 108 EO,
- (9*Z*,12*Z*)-Octadeca-9,12-dien-1-ol (Linoleylalkohol), ethoxyliert mit 109 EO,
- (9*Z*,12*Z*)-Octadeca-9,12-dien-1-ol (Linoleylalkohol), ethoxyliert mit 110 EO,
- (9*Z*,12*Z*)-Octadeca-9,12-dien-1-ol (Linoleylalkohol), ethoxyliert mit 111 EO,
- (9*Z*,12*Z*)-Octadeca-9,12-dien-1-ol (Linoleylalkohol), ethoxyliert mit 112 EO,
- (9*Z*,12*Z*)-Octadeca-9,12-dien-1-ol (Linoleylalkohol), ethoxyliert mit 113 EO,
- (9*Z*,12*Z*)-Octadeca-9,12-dien-1-ol (Linoleylalkohol), ethoxyliert mit 114 EO,
- (9*Z*,12*Z*)-Octadeca-9,12-dien-1-ol (Linoleylalkohol), ethoxyliert mit 115 EO,
- (9*Z*,12*Z*)-Octadeca-9,12-dien-1-ol (Linoleylalkohol), ethoxyliert mit 116 EO,
- (9*Z*,12*Z*)-Octadeca-9,12-dien-1-ol (Linoleylalkohol), ethoxyliert mit 117 EO,
- (9*Z*,12*Z*)-Octadeca-9,12-dien-1-ol (Linoleylalkohol), ethoxyliert mit 118 EO,
- (9*Z*,12*Z*)-Octadeca-9,12-dien-1-ol (Linoleylalkohol), ethoxyliert mit 119 EO,
- (9*Z*,12*Z*)-Octadeca-9,12-dien-1-ol (Linoleylalkohol), ethoxyliert mit 120 EO,
- Eicosan-1-ol (Eicosylalkohol, Arachylalkohol), ethoxyliert mit 80 EO,
- Eicosan-1-ol (Eicosylalkohol, Arachylalkohol), ethoxyliert mit 81 EO,
- Eicosan-1-ol (Eicosylalkohol, Arachylalkohol), ethoxyliert mit 82 EO,
- Eicosan-1-ol (Eicosylalkohol, Arachylalkohol), ethoxyliert mit 83 EO,
- Eicosan-1-ol (Eicosylalkohol, Arachylalkohol), ethoxyliert mit 84 EO,
- Eicosan-1-ol (Eicosylalkohol, Arachylalkohol), ethoxyliert mit 85 EO,
- Eicosan-1-ol (Eicosylalkohol, Arachylalkohol), ethoxyliert mit 86 EO,
- Eicosan-1-ol (Eicosylalkohol, Arachylalkohol), ethoxyliert mit 87 EO,
- Eicosan-1-ol (Eicosylalkohol, Arachylalkohol), ethoxyliert mit 88 EO,
- Eicosan-1-ol (Eicosylalkohol, Arachylalkohol), ethoxyliert mit 89 EO,
- Eicosan-1-ol (Eicosylalkohol, Arachylalkohol), ethoxyliert mit 90 EO,
- Eicosan-1-ol (Eicosylalkohol, Arachylalkohol), ethoxyliert mit 91 EO,
- Eicosan-1-ol (Eicosylalkohol, Arachylalkohol), ethoxyliert mit 92 EO,
- Eicosan-1-ol (Eicosylalkohol, Arachylalkohol), ethoxyliert mit 93 EO,
- Eicosan-1-ol (Eicosylalkohol, Arachylalkohol), ethoxyliert mit 94 EO,
- Eicosan-1-ol (Eicosylalkohol, Arachylalkohol), ethoxyliert mit 95 EO,
- Eicosan-1-ol (Eicosylalkohol, Arachylalkohol), ethoxyliert mit 96 EO,
- Eicosan-1-ol (Eicosylalkohol, Arachylalkohol), ethoxyliert mit 97 EO,
- Eicosan-1-ol (Eicosylalkohol, Arachylalkohol), ethoxyliert mit 98 EO,
- Eicosan-1-ol (Eicosylalkohol, Arachylalkohol), ethoxyliert mit 99 EO,
- Eicosan-1-ol (Eicosylalkohol, Arachylalkohol), ethoxyliert mit 100 EO,
- Eicosan-1-ol (Eicosylalkohol, Arachylalkohol), ethoxyliert mit 101 EO,
- Eicosan-1-ol (Eicosylalkohol, Arachylalkohol), ethoxyliert mit 102 EO,
- Eicosan-1-ol (Eicosylalkohol, Arachylalkohol), ethoxyliert mit 103 EO,
- Eicosan-1-ol (Eicosylalkohol, Arachylalkohol), ethoxyliert mit 104 EO,
- Eicosan-1-ol (Eicosylalkohol, Arachylalkohol), ethoxyliert mit 105 EO,
- Eicosan-1-ol (Eicosylalkohol, Arachylalkohol), ethoxyliert mit 106 EO,
- Eicosan-1-ol (Eicosylalkohol, Arachylalkohol), ethoxyliert mit 107 EO,
- Eicosan-1-ol (Eicosylalkohol, Arachylalkohol), ethoxyliert mit 108 EO,
- Eicosan-1-ol (Eicosylalkohol, Arachylalkohol), ethoxyliert mit 109 EO,
- Eicosan-1-ol (Eicosylalkohol, Arachylalkohol), ethoxyliert mit 110 EO,
- Eicosan-1-ol (Eicosylalkohol, Arachylalkohol), ethoxyliert mit 111 EO,
- Eicosan-1-ol (Eicosylalkohol, Arachylalkohol), ethoxyliert mit 112 EO,
- Eicosan-1-ol (Eicosylalkohol, Arachylalkohol), ethoxyliert mit 113 EO,
- Eicosan-1-ol (Eicosylalkohol, Arachylalkohol), ethoxyliert mit 114 EO,
- Eicosan-1-ol (Eicosylalkohol, Arachylalkohol), ethoxyliert mit 115 EO,
- Eicosan-1-ol (Eicosylalkohol, Arachylalkohol), ethoxyliert mit 116 EO,
- Eicosan-1-ol (Eicosylalkohol, Arachylalkohol), ethoxyliert mit 117 EO,
- Eicosan-1-ol (Eicosylalkohol, Arachylalkohol), ethoxyliert mit 118 EO,
- Eicosan-1-ol (Eicosylalkohol, Arachylalkohol), ethoxyliert mit 119 EO,
- Eicosan-1-ol (Eicosylalkohol, Arachylalkohol), ethoxyliert mit 120 EO,
- Docosan-1-ol (Docosylalkohol, Behenylalkohol), ethoxyliert mit 80 EO
- Docosan-1-ol (Docosylalkohol, Behenylalkohol), ethoxyliert mit 81 EO
- Docosan-1-ol (Docosylalkohol, Behenylalkohol), ethoxyliert mit 82 EO
- Docosan-1-ol (Docosylalkohol, Behenylalkohol), ethoxyliert mit 83 EO
- Docosan-1-ol (Docosylalkohol, Behenylalkohol), ethoxyliert mit 84 EO
- Docosan-1-ol (Docosylalkohol, Behenylalkohol), ethoxyliert mit 85 EO
- Docosan-1-ol (Docosylalkohol, Behenylalkohol), ethoxyliert mit 86 EO
- Docosan-1-ol (Docosylalkohol, Behenylalkohol), ethoxyliert mit 87 EO
- Docosan-1-ol (Docosylalkohol, Behenylalkohol), ethoxyliert mit 88 EO
- Docosan-1-ol (Docosylalkohol, Behenylalkohol), ethoxyliert mit 89 EO
- Docosan-1-ol (Docosylalkohol, Behenylalkohol), ethoxyliert mit 90 EO
- Docosan-1-ol (Docosylalkohol, Behenylalkohol), ethoxyliert mit 91 EO
- Docosan-1-ol (Docosylalkohol, Behenylalkohol), ethoxyliert mit 92 EO
- Docosan-1-ol (Docosylalkohol, Behenylalkohol), ethoxyliert mit 93 EO
- Docosan-1-ol (Docosylalkohol, Behenylalkohol), ethoxyliert mit 94 EO
- Docosan-1-ol (Docosylalkohol, Behenylalkohol), ethoxyliert mit 95 EO
- Docosan-1-ol (Docosylalkohol, Behenylalkohol), ethoxyliert mit 96 EO
- Docosan-1-ol (Docosylalkohol, Behenylalkohol), ethoxyliert mit 97 EO
- Docosan-1-ol (Docosylalkohol, Behenylalkohol), ethoxyliert mit 98 EO
- Docosan-1-ol (Docosylalkohol, Behenylalkohol), ethoxyliert mit 99 EO
- Docosan-1-ol (Docosylalkohol, Behenylalkohol), ethoxyliert mit 100 EO
- Docosan-1-ol (Docosylalkohol, Behenylalkohol), ethoxyliert mit 101 EO
- Docosan-1-ol (Docosylalkohol, Behenylalkohol), ethoxyliert mit 102 EO
- Docosan-1-ol (Docosylalkohol, Behenylalkohol), ethoxyliert mit 103 EO
- Docosan-1-ol (Docosylalkohol, Behenylalkohol), ethoxyliert mit 104 EO
- Docosan-1-ol (Docosylalkohol, Behenylalkohol), ethoxyliert mit 105 EO
- Docosan-1-ol (Docosylalkohol, Behenylalkohol), ethoxyliert mit 106 EO
- Docosan-1-ol (Docosylalkohol, Behenylalkohol), ethoxyliert mit 107 EO
- Docosan-1-ol (Docosylalkohol, Behenylalkohol), ethoxyliert mit 108 EO
- Docosan-1-ol (Docosylalkohol, Behenylalkohol), ethoxyliert mit 109 EO
- Docosan-1-ol (Docosylalkohol, Behenylalkohol), ethoxyliert mit 110 EO
- Docosan-1-ol (Docosylalkohol, Behenylalkohol), ethoxyliert mit 111 EO
- Docosan-1-ol (Docosylalkohol, Behenylalkohol), ethoxyliert mit 112 EO
- Docosan-1-ol (Docosylalkohol, Behenylalkohol), ethoxyliert mit 113 EO
- Docosan-1-ol (Docosylalkohol, Behenylalkohol), ethoxyliert mit 114 EO
- Docosan-1-ol (Docosylalkohol, Behenylalkohol), ethoxyliert mit 115 EO
- Docosan-1-ol (Docosylalkohol, Behenylalkohol), ethoxyliert mit 116 EO
- Docosan-1-ol (Docosylalkohol, Behenylalkohol), ethoxyliert mit 117 EO
- Docosan-1-ol (Docosylalkohol, Behenylalkohol), ethoxyliert mit 118 EO
- Docosan-1-ol (Docosylalkohol, Behenylalkohol), ethoxyliert mit 119 EO
- Docosan-1-ol (Docosylalkohol, Behenylalkohol), ethoxyliert mit 120 EO
eingesetzt werden.

Ein ganz besonders vorteilhaftes und damit explizit ganz besonders bevorzugtes Mittel zum Glätten, permanenten Umformen oder zur Depilation von keratinischen Fasern ist dadurch gekennzeichnet, dass es als ethoxylierte(n) Fettalkohol(e) eine oder mehrere Verbindungen aus der Gruppe
- Hexadecan-1-ol (Hexadecylalkohol, Cetylalkohol, Palmitylalkohol), ethoxyliert mit 90 EO,
- Hexadecan-1-ol (Hexadecylalkohol, Cetylalkohol, Palmitylalkohol), ethoxyliert mit 91 EO,
- Hexadecan-1-ol (Hexadecylalkohol, Cetylalkohol, Palmitylalkohol), ethoxyliert mit 92 EO,
- Hexadecan-1-ol (Hexadecylalkohol, Cetylalkohol, Palmitylalkohol), ethoxyliert mit 93 EO,
- Hexadecan-1-ol (Hexadecylalkohol, Cetylalkohol, Palmitylalkohol), ethoxyliert mit 94 EO,
- Hexadecan-1-ol (Hexadecylalkohol, Cetylalkohol, Palmitylalkohol), ethoxyliert mit 95 EO,
- Hexadecan-1-ol (Hexadecylalkohol, Cetylalkohol, Palmitylalkohol), ethoxyliert mit 96 EO,
- Hexadecan-1-ol (Hexadecylalkohol, Cetylalkohol, Palmitylalkohol), ethoxyliert mit 97 EO,
- Hexadecan-1-ol (Hexadecylalkohol, Cetylalkohol, Palmitylalkohol), ethoxyliert mit 98 EO,
- Hexadecan-1-ol (Hexadecylalkohol, Cetylalkohol, Palmitylalkohol), ethoxyliert mit 99 EO,
- Hexadecan-1-ol (Hexadecylalkohol, Cetylalkohol, Palmitylalkohol), ethoxyliert mit 100 EO,
- Hexadecan-1-ol (Hexadecylalkohol, Cetylalkohol, Palmitylalkohol), ethoxyliert mit 101 EO,
- Hexadecan-1-ol (Hexadecylalkohol, Cetylalkohol, Palmitylalkohol), ethoxyliert mit 102 EO,
- Hexadecan-1-ol (Hexadecylalkohol, Cetylalkohol, Palmitylalkohol), ethoxyliert mit 103 EO,
- Hexadecan-1-ol (Hexadecylalkohol, Cetylalkohol, Palmitylalkohol), ethoxyliert mit 104 EO,
- Hexadecan-1-ol (Hexadecylalkohol, Cetylalkohol, Palmitylalkohol), ethoxyliert mit 105 EO,
- Hexadecan-1-ol (Hexadecylalkohol, Cetylalkohol, Palmitylalkohol), ethoxyliert mit 106 EO,
- Hexadecan-1-ol (Hexadecylalkohol, Cetylalkohol, Palmitylalkohol), ethoxyliert mit 107 EO,
- Hexadecan-1-ol (Hexadecylalkohol, Cetylalkohol, Palmitylalkohol), ethoxyliert mit 108 EO,
- Hexadecan-1-ol (Hexadecylalkohol, Cetylalkohol, Palmitylalkohol), ethoxyliert mit 109 EO,
- Hexadecan-1-ol (Hexadecylalkohol, Cetylalkohol, Palmitylalkohol), ethoxyliert mit 110 EO,
- Octadecan-1-ol (Octadecylalkohol, Stearylalkohol), ethoxyliert mit 90 EO,
- Octadecan-1-ol (Octadecylalkohol, Stearylalkohol), ethoxyliert mit 91 EO,
- Octadecan-1-ol (Octadecylalkohol, Stearylalkohol), ethoxyliert mit 92 EO,
- Octadecan-1-ol (Octadecylalkohol, Stearylalkohol), ethoxyliert mit 93 EO,
- Octadecan-1-ol (Octadecylalkohol, Stearylalkohol), ethoxyliert mit 94 EO,
- Octadecan-1-ol (Octadecylalkohol, Stearylalkohol), ethoxyliert mit 95 EO,
- Octadecan-1-ol (Octadecylalkohol, Stearylalkohol), ethoxyliert mit 96 EO,
- Octadecan-1-ol (Octadecylalkohol, Stearylalkohol), ethoxyliert mit 97 EO,
- Octadecan-1-ol (Octadecylalkohol, Stearylalkohol), ethoxyliert mit 98 EO,
- Octadecan-1-ol (Octadecylalkohol, Stearylalkohol), ethoxyliert mit 99 EO,
- Octadecan-1-ol (Octadecylalkohol, Stearylalkohol), ethoxyliert mit 100 EO,
- Octadecan-1-ol (Octadecylalkohol, Stearylalkohol), ethoxyliert mit 101 EO,
- Octadecan-1-ol (Octadecylalkohol, Stearylalkohol), ethoxyliert mit 102 EO,
- Octadecan-1-ol (Octadecylalkohol, Stearylalkohol), ethoxyliert mit 103 EO,
- Octadecan-1-ol (Octadecylalkohol, Stearylalkohol), ethoxyliert mit 104 EO,
- Octadecan-1-ol (Octadecylalkohol, Stearylalkohol), ethoxyliert mit 105 EO,
- Octadecan-1-ol (Octadecylalkohol, Stearylalkohol), ethoxyliert mit 106 EO,
- Octadecan-1-ol (Octadecylalkohol, Stearylalkohol), ethoxyliert mit 107 EO,
- Octadecan-1-ol (Octadecylalkohol, Stearylalkohol), ethoxyliert mit 108 EO,
- Octadecan-1-ol (Octadecylalkohol, Stearylalkohol), ethoxyliert mit 109 EO,
- Octadecan-1-ol (Octadecylalkohol, Stearylalkohol), ethoxyliert mit 110 EO,
enthält.

Besonders bevorzugte ethoxylierte Fettalkohole mit einem Ethoxylierungsgrad von 80 bis 120 sind ausgewählt aus der Gruppe
- Octadecan-1-ol (Octadecylalkohol, Stearylalkohol), ethoxyliert mit 90 EO,
- Octadecan-1-ol (Octadecylalkohol, Stearylalkohol), ethoxyliert mit 91 EO,
- Octadecan-1-ol (Octadecylalkohol, Stearylalkohol), ethoxyliert mit 92 EO,
- Octadecan-1-ol (Octadecylalkohol, Stearylalkohol), ethoxyliert mit 93 EO,
- Octadecan-1-ol (Octadecylalkohol, Stearylalkohol), ethoxyliert mit 94 EO,
- Octadecan-1-ol (Octadecylalkohol, Stearylalkohol), ethoxyliert mit 95 EO,
- Octadecan-1-ol (Octadecylalkohol, Stearylalkohol), ethoxyliert mit 96 EO,
- Octadecan-1-ol (Octadecylalkohol, Stearylalkohol), ethoxyliert mit 97 EO,
- Octadecan-1-ol (Octadecylalkohol, Stearylalkohol), ethoxyliert mit 98 EO,
- Octadecan-1-ol (Octadecylalkohol, Stearylalkohol), ethoxyliert mit 99 EO,
- Octadecan-1-ol (Octadecylalkohol, Stearylalkohol), ethoxyliert mit 100 EO,
- Octadecan-1-ol (Octadecylalkohol, Stearylalkohol), ethoxyliert mit 101 EO,
- Octadecan-1-ol (Octadecylalkohol, Stearylalkohol), ethoxyliert mit 102 EO,
- Octadecan-1-ol (Octadecylalkohol, Stearylalkohol), ethoxyliert mit 103 EO,
- Octadecan-1-ol (Octadecylalkohol, Stearylalkohol), ethoxyliert mit 104 EO,
- Octadecan-1-ol (Octadecylalkohol, Stearylalkohol), ethoxyliert mit 105 EO,
- Octadecan-1-ol (Octadecylalkohol, Stearylalkohol), ethoxyliert mit 106 EO,
- Octadecan-1-ol (Octadecylalkohol, Stearylalkohol), ethoxyliert mit 107 EO,
- Octadecan-1-ol (Octadecylalkohol, Stearylalkohol), ethoxyliert mit 108 EO,
- Octadecan-1-ol (Octadecylalkohol, Stearylalkohol), ethoxyliert mit 109 EO,
- Octadecan-1-ol (Octadecylalkohol, Stearylalkohol), ethoxyliert mit 110 EO.

In einer weiteren bevorzugten Ausführungsform ist ein Mittel zum Glätten, permanenten Umformen oder zur Depilation von keratinischen Fasern deshalb dadurch gekennzeichnet, dass es einen oder mehrere ethoxylierte Fettalkohole mit einem Ethoxylierungsgrad von 80 bis 120 in einer Gesamtmenge von 0,2 bis 1,5 Gew.-%, bevorzugt von 0,3 bis 1,2 Gew.-%, weiter bevorzugt von 0,4 bis 0,9 Gew.-% und besonders bevorzugt von 0,5 bis 0,8 Gew.-% - bezogen auf das Gewicht des Mittels - enthält.

Ebenfalls von Vorteil im Hinblick auf eine maximale Reduzierung des schwefligen Geruchs ist die Anwesenheit mindestens eines weiteren ethoxylierten Fettalkohols mit einem Ethoxylierungsgrad von 20 bis 40, der bevorzugt in einer Gesamtmenge von 0,1 bis 5 Gew.-%, besonders bevorzugt 0,5 bis 4 Gew.-%, außerordentlich bevorzugt 1,5 bis 3 Gew.-%, bezogen auf das Gewicht des Mittels, enthalten ist.

In einer weiteren ebenfalls bevorzugten Ausführungsform ist ein erfindungsgemäßes Mittel zum Glätten, permanenten Umformen oder zur Depilation von keratinischen Fasern deshalb dadurch gekennzeichnet, dass es zusätzlich ein oder mehrere ethoxylierte Fettalkohole mit einem Ethoxylierungsgrad von 20 bis 40 enthält. Geeignete Fettalkohole mit einem Ethoxylierungsgrad von 20 bis 40 sind
- Dodecan-1-ol (Dodecylalkohol, Laurylalkohol), ethoxyliert mit 20 bis 40 EO,
- Tetradecan-1-ol, (Tetradecylalkohol, Myristylalkohol), ethoxyliert mit 20 bis 40 EO,
- Hexadecan-1-ol (Hexadecylalkohol, Cetylalkohol, Palmitylalkohol), ethoxyliert mit 20 bis 40 EO,
- Hexadecan-1-ol (Hexadecylalkohol, Cetylalkohol, Palmitylalkohol), ethoxyliert mit 20 bis 40 EO,
- Octadecan-1-ol (Octadecylalkohol, Stearylalkohol), ethoxyliert mit 20 bis 40 EO,
- (9*Z*)-Octadec-9-en-1-ol (Oleylalkohol), ethoxyliert mit 20 bis 40 EO,
- (9*E*)-Octadec-9-en-1-ol (Elaidylalkohol), ethoxyliert mit 20 bis 40 EO,
- (9*Z*,12*Z*)-Octadeca-9,12-dien-1-ol (Linoleylalkohol), ethoxyliert mit 20 bis 40 EO,
- Eicosan-1-ol (Eicosylalkohol, Arachylalkohol), ethoxyliert mit 20 bis 40 EO,
- Docosan-1-ol (Docosylalkohol, Behenylalkohol), ethoxyliert mit 20 bis 40 EO
und deren Gemische.

Die ethoxylierten Fettalkohole mit einem Ethoxylierungsgrad von 80 bis 120 und die ethoxylierten Fettalkohole mit einem Ethoxylierungsgrad von 30 sind in einer vorteilhaften Ausführungsform in bestimmten Mengenbereichen und in speziellen Gewcihtsverhältnissen enthalten.

In einer weiteren ebenfalls bevorzugten Ausführungsform ist ein erfindungsgemäßes Mittel zum Glätten, permanenten Umformen oder zur Depilation von keratinischen Fasern schließlich dadurch gekennzeichnet, dass es die ethoxylierten Fettalkohole (III') mit einem Ethoxylierungsgrad von 30 und die ethoxylierten Fettalkohole (III) mit einem Ethoxylierungsgrad von 80 bis 120 in einem Gewichtsverhältnis (III') / (III) von mindestens 1:1, bevorzugt in einem Gewichtsverhältnis von mindestens 1,5:1 und besonders bevorzugt in einem Gewichtsverhältnis von mindestens 2:1 - jeweils bezogen auf die Gesamtmenge aller im erfindungsgemäßen Mittel enthaltenen ethoxylierten Fettalkohole (III') und die Gesamtmenge aller im erfindungsgemäßen Mittel enthaltenen ethoxylierten Fettalkohole (III) - enthält. In einer weiteren besonders bevorzugten Ausführungsform der Erfindung enthalten die erfindungsgemäßen Mittel ein oder mehrere zwitterionische Polymere.

Unter Polymeren werden Makromoleküle mit einem Molekulargewicht von mindestens 1000 g/mol, bevorzugt von mindestens 2500 g/mol, besonders bevorzugt von mindestens 5000 g/mol, verstanden, welche aus gleichen, sich wiederholenden organischen Einheiten bestehen. Polymere werden durch Polymerisation von einem Monomertyp oder durch Polymerisation von verschiedenen, strukturell voneinander unterschiedlichen Monomertypen hergestellt. Wird das Polymer durch Polymerisation eines Monomertyps hergestellt, spricht man von Homo-Polymeren. Werden strukturell unterschiedliche Monomertypen in der Polymerisation eingesetzt, spricht der Fachmann von Copolymeren.

Das maximale Molekulargewicht des Polymers hängt vom Polymerisationsgrad (Anzahl der polymerisierten Monomere) ab und wird durch die Polymerisationsmethode mitbestimmt. Im Sinne der vorliegenden Erfindung ist es bevorzugt, wenn das Molekulargewicht des zwitterionischen Polymers 100.000 bis 10⁷ g/mol, bevorzugt 200.000 bis 5·10⁶ g/mol und besonders bevorzugt 500.000 bis 1·10⁶ g/mol beträgt.

Unter zwitterionischen Polymeren werden solche Polymere verstanden, die im Makromolekül sowohl kationische Gruppierungen als auch anionische Gruppierungen enthalten. Bei den im Makromolekül enthaltenen kationischen Gruppierungen handelt es sich quartäre Ammoniumgruppen. Bei diesen quartären Ammoniumgruppen trägt ein positiv geladenes Stickstoffatom vier organische Reste. Bei den anionischen Gruppierungen handelt es sich um -COO⁻-Gruppen oder -SO₃⁻-Gruppen.

Um eine besonders dauerhafte und starke Minimierung des schwefligen Geruchs zu erzielen, ist es von besonderem Vorteil, auch die zwitterionischen Polymere in speziellen Mengenbereichen einzusetzen.

In einer weiteren besonders bevorzugten Ausführungsform ist ein Mittel zum Glätten, permanenten Umformen oder zur Depilation von keratinischen Fasern daher dadurch gekennzeichnet, dass es ein oder mehrere zwitterionische Polymere in einer Gesamtmenge von 0,1 bis 1,5 Gew.-%, bevorzugt von 0,2 bis 1,2 Gew.-%, weiter bevorzugt von 0,3 bis 0,8 Gew.-% und besonders bevorzugt von 0,4 bis 0,6 Gew.-% - bezogen auf das Gewicht des Mittels - enthält.

Bevorzugte zwitterionische Polymere sind ausgewählt aus der Gruppe der
- Copolymere aus Dimethyl-diallylammoniumsalzen und Acrylsäure, z.B. Polyquaternium-22,
- Copolymere aus Dimethyl-diallylammoniumsalzen und Methacrylsäure,
- Copolymere aus N,N,N-Trimethyl-3-[(2-methyl-1-oxo-2-propen-1-yl)amino]-1-propanaminiumsalzen und Acrylsäure,
- Copolymere aus N,N,N-Trimethyl-3-[(2-methyl-1-oxo-2-propen-1-yl)amino]-1-propanaminiumsalzen und Methacrylsäure,
- Copolymere aus N,N,N-Trimethyl-2-[(2-methyl-1-oxo-2-propen-1-yl)amino]-1-ethanaminiumsalzen und Acrylsäure,
- Copolymere aus N,N,N-Trimethyl-2-[(2-methyl-1-oxo-2-propen-1-yl)amino]-1-ethanaminiumsalzen und Methacrylsäure,
- Copolymere aus N,N,N-Trimethyl-3-[(2-methyl-1-oxo-2-propen-1-yl)amino]-1-propanaminiumsalzen, Acrylsäure und Acrylamid, z.B. Polyquaternium-53
- Copolymere aus N,N,N-Trimethyl-3-[(2-methyl-1-oxo-2-propen-1-yl)amino]-1-propanaminiumsalzen, Methacrylsäure und Acrylamid,
- Copolymere aus 1-Ethenyl-3-methyl-1H-imidazoliumsalzen, 1-Ethenyl-1H-imidazol, 1-Ethenyl-2-pyrrolidinon und Methacrylsäure, z.B. Polyquaternium-86,
- Copolymere aus 1-Ethenyl-3-methyl-1H-imidazoliumsalzen, 1-Ethenyl-1H-imidazol, 1-Ethenyl-2-pyrrolidinon und Acrylsäure.

Auch Gemische der vorgenannten bevorzugten zwitterionischen Polymere können in den erfindungsgemäß besonders bevorzugten Mitteln enthalten sein.

Innerhalb der Gruppe der bevorzugten zwitterionischen Polymere besitzen ganz bestimmte zwitterionische Polymere eine besonders herausragende Eignung zur Reduzierung des schwefligen Geruchs. Bei Einsatz dieser ausgewählten Polymere wird nicht nur die geruchliche Wahrnehmung erfindungsgemäß bevorzugter Mittel optimiert, sondern gleichzeitig auch das Well-, Glätt- oder Depilationsergebnis verbessert.

Aus diesem Grund ist in einer weiteren explizit ganz besonders bevorzugten Ausführungsform ein Mittel zum Glätten, permanenten Umformen oder zur Depilation von keratinischen Fasern dadurch gekennzeichnet, dass es ein oder mehrere zwitterionische Polymere enthält, die mindestens eine anionische Struktureinheit der Formel (IV) und mindestens eine kationische Struktureinheit der Formel (V) enthalten, 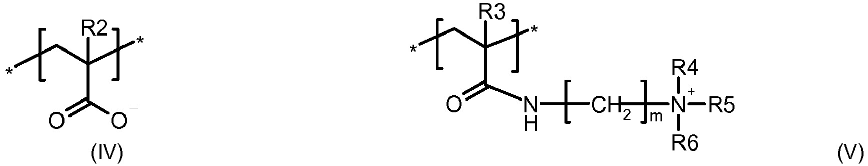
worin R2 und R3 unabhängig voneinander für ein Wasserstoffatom oder eine Methylgruppe stehen,
m für eine ganze Zahl von 2 bis 6, bevorzugt für die Zahlen 2 oder 3, steht und
die Reste R4, R5 und R6 unabhängig voneinander für eine C₁-C₆-Alkylgruppe, bevorzugt unabhängig voneinander für eine Methylgruppe, eine Ethylgruppe oder eine Propylgruppe, stehen.

Ein besonders bevorzugtes zwitterionisches Polymer dieses Typs ist unter dem INCI-Namen Acrylamidopropyltrimonium chloride /Acrylate Copolymer bekannt.

In einer weiteren besonders bevorzugten Ausführungsform ist ein Mittel zum Glätten, permanenten Umformen oder zur Depilation von keratinischen Fasern daher dadurch gekennzeichnet, dass es in einer Gesamtmenge von 0,1 bis 1,5 Gew.-%, bevorzugt von 0,2 bis 1,2 Gew.-%, weiter bevorzugt von 0,3 bis 0,8 Gew.-% und besonders bevorzugt von 0,4 bis 0,6 Gew.-% - bezogen auf das Gewicht des Mittels - ein oder mehrere zwitterionische Polymere enthält, die mindestens eine anionische Struktureinheit der Formel (IV) und mindestens eine kationische Struktureinheit der Formel (V) enthalten.

In einer weiteren ebenfalls besonders bevorzugten Ausführungsform ist ein erfindungsgemäßes Mittel zum Glätten, permanenten Umformen oder zur Depilation von keratinischen Fasern dadurch gekennzeichnet, dass es ein oder mehrere zwitterionische Polymere enthält, die mindestens eine anionische Struktureinheit der Formel (VI) und mindestens eine kationische Struktureinheit der Formel (VII) enthalten, 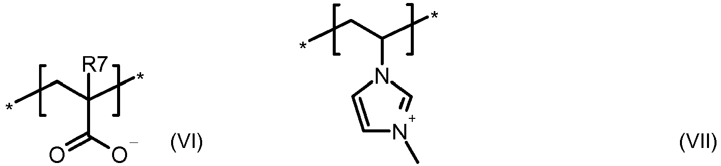 worin R7 für ein Wasserstoffatom oder eine Methylgruppe steht.

In einer weiteren besonders bevorzugten Ausführungsform ist ein Mittel zum Glätten, permanenten Umformen oder zur Depilation von keratinischen Fasern daher dadurch gekennzeichnet, dass es in einer Gesamtmenge von 0,1 bis 1,5 Gew.-%, bevorzugt von 0,2 bis 1,2 Gew.-%, weiter bevorzugt von 0,3 bis 0,8 Gew.-% und besonders bevorzugt von 0,4 bis 0,6 Gew.-% - bezogen auf das Gewicht des Mittels - ein oder mehrere zwitterionische Polymere enthält, die mindestens eine anionische Struktureinheit der Formel (VI) und mindestens eine kationische Struktureinheit der Formel (VII) enthalten.

In einer weiteren besonders bevorzugten Ausführungsform ist ein Mittel zum Glätten, permanenten Umformen oder zur Depilation von keratinischen Fasern daher dadurch gekennzeichnet, dass es in einer Gesamtmenge von 0,1 bis 1,5 Gew.-%, bevorzugt von 0,2 bis 1,2 Gew.-%, weiter bevorzugt von 0,3 bis 0,8 Gew.-% und besonders bevorzugt von 0,4 bis 0,6 Gew.-% - bezogen auf das Gewicht des Mittels - N,N,N-Trimethyl-3-[(1-oxo-2-propenyl)amino]-1-propanaminiumchloride, polymer with sodium 2-propenoate mit der INCI Acrylamidopropyltrimonium chloride/Acrylate Copolymer als zwitterionisches Polymer enthält.

Zur Einstellung dieses pH-Wertes enthalten die erfindungsgemäßen Well- Glätt- oder Depiliermittel üblicherweise Alkalisierungsmittel wie Ammoniak, Alkali- und Ammonium-carbonate und -hydrogencarbonate oder organische Amine, insbesondere Alkanolamine, wie Monoethanolamin.

Bevorzugt wird Ammoniak in Form seiner wässrigen Lösung eingesetzt. Bei entsprechenden wässrigen Ammoniak-Lösungen kann es sich um 10 bis 35 prozentige Lösungen handeln (berechnet in Gew.-%, 100 g wässrige Ammoniaklösung enthalten dementsprechend 10 bis 35 g Ammoniak). Bevorzugt wird Ammoniak in Form einer 20 bis 30 Gew.-%igen Lösung, besonders bevorzugt in Form einer 25 Gew.-%igen Lösung eingesetzt. In einer weiteren besonders bevorzugten Ausführungsform ist ein Mittel zum Glätten, permanenten Umformen oder zur Depilation von keratinischen Fasern daher dadurch gekennzeichnet, dass es Ammoniak in einer Menge von 0,25 bis 1,75 Gew.-%, bevorzugt von 0,4 bis 1,4 Gew.-%, weiter bevorzugt von 0,6 bis 1,1 Gew.-% und besonders bevorzugt von 0,7 bis 0,9 Gew.-% - bezogen auf das Gewicht des Mittels - enthält.

Bei Alkanolaminen handelt es sich um primäre, sekundäre oder tertiäre Amine mit einem C₂-C₆-Alkylgrundkörper, der mindestens eine Hydroxylgruppe trägt. Beispielhaft als Alkanolamine können 2-Aminoethan-1-ol (Monoethanolamin), 2-Amino-2-methylpropan-1-ol, 2-Amino-2-methyl-propan-1,3-diol und Triethanolamin genannt werden.

Die Fixierung der umgeformten Keratinfasern erfolgt erst durch den Einfluss eines Oxidationsmittels. Üblicherweise wird hierfür Wasserstoffperoxid verwendet. In einer bevorzugten Ausführungsform wird das Wasserstoffperoxid als wässrige Lösung verwendet. Erfindungsgemäß bevorzugte Oxidationsmittelzubereitungen sind dadurch gekennzeichnet, dass sie 1,0 bis 23,0 Gew.-%, weiter bevorzugt 2,5 bis 21,0 Gew.-%, besonders bevorzugt 4,0 bis 20,0 Gew.-% und ganz besonders bevorzugt 5,0 bis 18,0 Gew.-% Wasserstoffperoxid (berechnet als 100 %-iges H₂O₂) enthalten.

Es hat sich als vorteilhaft erwiesen, wenn die erfindungsgemäßen Oxidationsmittelzubereitungen zur Stabilisierung des Wasserstoffperoxids zusätzlich mindestens einen Stabilisator oder Komplexbildner enthalten. Besonders bevorzugte Stabilisatoren sind insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Zusätzlich zu den zwitterionischen Polymeren können erfindungsgemäß bevorzugte Mittel auch anionische Polymere enthalten. Beispiele für geeignete anionische Polymere sind beispielsweise unter dem Warenzeichnen Carbopol® oder Rheothik®11-80 im Handel erhältlich. Auch die Polymere, die unter dem INCI-Namen Acrylates Copolymere vertrieben werden, sind geeignete anionische Polymere. Ein bevorzugtes Handelsprodukt ist beispielsweise Aculyn® 33 der Firma Dow Corning (vormals von Rohm & Haas). Weitere bevorzugte anionische Polymere werden von der Firma Dow Corning (vormals von Rohm & Haas) unter der Handelsbezeichnung Aculyn® 22 sowie von der Firma AkzoNobel unter den Handelsbezeichnungen Structure® 2001 und Structure® 3001 vertrieben.

Geeignete zusätzlich einsetzbare kationische Polymere sind beispielsweise Polyquaternium-24 (Handelsprodukt z. B. Quatrisoft® LM 200), Copolymer 845 (Hersteller: ISP), Gaffix® VC 713 (Hersteller: ISP), Gafquat®ASCP 1011, Gafquat®HS 110, Luviquat®8155 und Luviquat® MS 370.

Auch natürlich vorkommende Verdickungsmittel können auch nichtionische Guargums, wie beispielsweise sowohl modifizierte (z.B. Jaguar® HP8, Jaguar® HP60, Jaguar® HP120, Jaguar® DC 293 und Jaguar® HP105) als auch unmodifizierte Guargums (z.B. Jaguar® C) zum Einsatz kommen. Weitere geeignete Verdickungsmittel sind die Skleroglucangums oder Xanthangums, Gums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen.

Weitere anionische, kationische oder amphotere Tenside können ebenfalls in den erfindungsgemäßen Mitteln enthalten sein. Bevorzugte anionische Tenside sind Alkylsulfate, Alkylethersulfate und Ethercarbonsäuren mit 10 bis 18 C-Atomen in der Alkylgruppe und bis zu 12 Glykolethergruppen im Molekül. Besonders bevorzugte amphotere Tenside sind das N-Kokosalkylaminopropionat, das Kokosacylaminoethylaminopropionat und das C₁₂-C₁₈-Acylsarcosin.

Bevorzugte zusätzlich enthaltene kationische Tenside sind beispielsweise Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen.

Ferner können die erfindungsgemäßen Mittel weitere Wirk-, Hilfs- und Zusatzstoffe enthalten, wie beispielsweise nichtionische Polymere, Silikone, kationische Polymere, Strukturanden, Lösungsmittel und Vermittler, faserstrukturverbessernde Wirkstoffe, Entschäumer wie Silikone, Antischuppenwirkstoffe, Proteinhydrolysate, pflanzliche Öle, beispielsweise Macadamianussöl, Kukuinussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl und Teebaumöl, Lichtschutzmittel, Substanzen zur Einstellung des pH-Werts, wie beispielsweise übliche Säuren, insbesondere Genusssäuren, Vitamine, Provitamine und Vitaminvorstufen, Pflanzenextrakte, Konsistenzgeber, Wachse, weitere Quell- und Penetrationsstoffe, Perlglanzmittel wie Ethylenglykolmono- und -distearat sowie PEG-3-distearat, Treibmittel wie Propan-Butan-Gemische, N₂O, Dimethylether, CO₂ und Luft und Antioxidantien.

Die Auswahl dieser weiteren Stoffe wird der Fachmann gemäß der gewünschten Eigenschaften der Mittel treffen.

Die zusätzlichen Wirk- und Hilfsstoffe sind in den erfindungsgemäßen Mitteln bevorzugt in Mengen von jeweils 0,0001 bis 10 Gew.-%, insbesondere von 0,0005 bis 5 Gew.-%, bezogen auf das Gewicht des Mittels, enthalten.

Umformprozesse auf Keratinfasern sowie die Depilation laufen üblicherweise im alkalischen oder schwach sauren Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des erfindungsgemäßen Mittels im Bereich von 5 bis 13 liegt. Besonders bevorzugte erfindungsgemäße Glättungs- und Wellmittel weisen einen pH-Wert im Bereich von 7 bis 9,5 auf.

Besonders bevorzugte erfindungsgemäße Depiliermittel weisen einen pH-Wert im Bereich von 10,5 bis 12,7 auf. Bei den pH-Werten im Sinne der vorliegenden Erfindung handelt es sich um pH-Werte, die bei einer Temperatur von 22 °C gemessen wurden.

Ein weiterer Gegenstand der vorliegenden Erfindung ist die Verwendung einer Kombination aus
(a) mindestens einem linearen Alkan-1-ol oder Alken-1-ol mit jeweils 20 bis 22 Kohlenstoffatomen, wobei das mindestens eine Alken-1-ol eine bis vier olefinische Doppelbindungen aufweist, in einer Gesamtmenge von 1,8 bis 10,0 Gew.-%, bezogen auf das Gewicht des Mittels, weiter
(b) mindestens einem Glycerylfettsäureester der allgemeinen Formel (I), 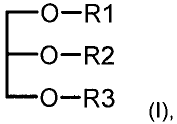 worin
   R1, R2 und R3 unabhängig voneinander für ein Wasserstoffatom oder eine Gruppierung der Formel (II) stehen, 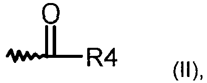 worin
   R4 für eine unverzweigte oder verzweigte, gesättigte oder ungesättigte C₁₁-C₂₇-Alkylgruppe steht,
   mit der Maßgabe, dass mindestens einer und maximal zwei der Reste ausgewählt aus R1, R2 und R3 für eine Gruppierung der Formel (II) stehen, und
(c) Wasser
   in Mitteln zum Glätten, permanenten Umformen oder zur Depilation von Haaren, enthaltend mindestens eine Keratin reduzierende Verbindung, ausgewählt aus Thioglykolsäure und deren Salzen und/oder Thiomilchsäure und deren Salzen und/oder Cystein und/oder Acetylcystein und/oder Cysteamin und deren Salzen sowie Mischungen dieser Keratin reduzierenden Verbindungen, zur Reduktion des Geruchs der mindestens einen Keratin reduzierenden Verbindung vor, während und nach dem Glätt-, Umformungs- oder Depilationsvorgang,
dadurch gekennzeichnet, dass der mindestens eine Glycerylfettsäureester (b) der allgemeinen Formel (I) in einer Gesamtmenge von 0,1 bis 0,8 Gew.-% - bezogen auf das Gewicht des Mittels - enthalten ist.

Ein weiterer Gegenstand der vorliegenden Erfindung ist ein Verfahren zum Glätten, permanenten
Umformen oder zur Depilation von keratinischen Fasern, in welchem
(i) die Fasern unter Zuhilfenahme von mechanischen Verformungshilfsmitteln, wie Wickler oder Papilloten, nach, vor oder während des Schritts (ii) verformt werden,
(ii) ein Mittel nach einem der Ansprüche 1 bis 12 auf die Fasern aufgetragen wird,
(iii) die Fasern nach einer Einwirkzeit Z1, bevorzugt nach 5-60 Minuten, besonders bevorzugt nach 10-30 Minuten gespült und gegebenenfalls getrocknet werden,
(iv) anschließend ein Fixiermittel, enthaltend mindestens ein Oxidationsmittel, auf die Fasern aufgetragen und nach einer Einwirkzeit Z2, bevorzugt nach 1-30 Minuten, besonders bevorzugt nach 5-20 Minuten wieder abgespült wird.

Bezüglich weiterer bevorzugter Ausführungsformen der erfindungsgemäßen Verfahren und Verwendungen gilt mutatis mutandis das zu den erfindungsgemäßen Mitteln Gesagte.

### Beispielteil

Folgende Glättungsmittel wurden hergestellt (alle Mengenangaben in Gew.-%):

| | | erfindungsgemäßes Beispiel | Vergleichsbeispiel |
|---|---|---|---|
| | | | |
| Cetearyl Alcohol (Cetylalkohol und Stearylalkohol im Gewichtsverhältnis 1:1) | | - | 7,000000 |
| Cetylalkohol | | 3,600000 | - |
| Lanette 22 | | 2,400000 | 1,000000 |
| 1,2-Propylenglycol | | 6,000000 | 1,000000 |
| Eumulgin B 3 | | 1,200000 | 0,500000 |
| Cutina GMS-V | | 0,600000 | 1,000000 |
| Hydroxyethylcellulose | | - | 0,300000 |
| Ammoniumthioglycolat | (71 Gew.-% in Wasser) | 9,200000 | 9,200000 |
| HEDP | (60 Gew.-% in Wasser) | 0,300000 | 0,300000 |
| Ammoniak | (25 Gew.-% in Wasser) | 1,200000 | 1,200000 |
| Parfum | | 0,700000 | 0,700000 |
| Ammoniumhydrogencarbonat | | 3,000000 | 3,000000 |
| Paraffinum Liquidum | | 2,100000 | - |
| Brij S100-PA | | 0,600000 | - |
| Product W 37194 | | 1,250000 | - |
| Water, demineralized | | ad 100,000000 | ad 100,000000 |

Beide Glättungsmittel wurden von Parfumeuren abgerochen.

Das erfindungsgemäße Beispiel zeigte einen deutlich schwächeren schwefligen Geruch nach Thioglycolsäure als das Vergleichsbeispiel.

**Liste der eingesetzten Rohstoffe**

| Produktname | INCI | | Hersteller |
|---|---|---|---|
| Brij S100-PA | Steareth-100 | | Croda |
| Cutina GMS-V | Glyceryl Stearate | Glycerylmonostearat und Glyceryldistearat im Gewichtsverhältnis 1:1 | BASF |
| Eumulgin B 3 | Ceteareth-30 | | BASF |
| Lanette 22 | Behenyl alcohol | C18:0-Alkohol: 5-15 Gew.-%, C20:0-Alkohol: 10-20 Gew.-%, C22:0-Alkohol: 70-80 Gew.-% | BASF |
| HEDP, 60 Gew.-% | Etidronic acid, Aqua (Water) | | |
| Product W 37194 | Acrylamidopropyltrimonium chloride/Acrylates Copolymer, Aqua (Water) | Sodium acrylate, trimethylammoniopropylacrylamide chloride copolymer (19-21 Gew.-% in Wasser) | Bozzetto GmbH (Stockhausen) |

## Patentansprüche

1. Mittel zum Glätten, permanenten Umformen oder zur Depilation von keratinischen Fasern, insbesondere menschlichen Haaren, enthaltend
(a) mindestens ein lineares Alkan-1-ol oder Alken-1-ol mit jeweils 20 bis 22 Kohlenstoffatomen, wobei das mindestens eine Alken-1-ol eine bis vier olefinische Doppelbindungen aufweist, in einer Gesamtmenge von 1,8 bis 10,0 Gew.-%, bezogen auf das Gewicht des Mittels, weiter
(b) mindestens einen Glycerylfettsäureester der allgemeinen Formel (I), 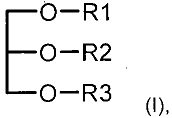 worin
R1, R2 und R3 unabhängig voneinander für ein Wasserstoffatom oder eine Gruppierung der Formel (II) stehen, 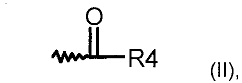 worin
R4 für eine unverzweigte oder verzweigte, gesättigte oder ungesättigte C₁₁-C₂₇-Alkylgruppe steht, mit der Maßgabe, dass mindestens einer und maximal zwei der Reste ausgewählt aus R1, R2 und R3 für eine Gruppierung der Formel (II) stehen, weiterhin
(c) Wasser und
(d) mindestens eine Keratin reduzierende Verbindung, ausgewählt aus Thioglykolsäure und deren Salzen und / oder Thiomilchsäure und deren Salzen und/oder Cystein und/oder Acetylcystein und/oder Cysteamin und deren Salzen sowie Mischungen dieser Keratin reduzierenden Verbindungen,
**dadurch gekennzeichnet, dass** der mindestens eine Glycerylfettsäureester (b) der allgemeinen Formel (I) in einer Gesamtmenge von 0,1 bis 0,8 Gew.-% - bezogen auf das Gewicht des Mittels - enthalten ist.

2. Mittel nach Anspruch 1, **dadurch gekennzeichnet, dass** die mindestens eine Keratin reduzierende Verbindung (d) in einer Gesamtmenge von 0,1 bis 20 Gew.%, bevorzugt 1 bis 15 Gew.-%, besonders bevorzugt 3 bis 13 Gew.-%, jeweils bezogen auf das Gewicht des Mittels, enthalten ist.

3. Mittel nach Anspruch 1 oder 2, **dadurch gekennzeichnet, dass** das mindestens eine lineare Alkan-1-ol oder Alken-1-ol mit jeweils 20 bis 22 Kohlenstoffatomen ausgewählt ist aus Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol) sowie Mischungen hiervon, insbesondere ausgewählt aus Mischungen von Arachylalkohol und Behenylalkohol.

4. Mittel nach einem der Ansprüche 1 bis 3, **dadurch gekennzeichnet, dass** das mindestens eine lineare Alkan-1-ol oder Alken-1-ol mit jeweils 20 bis 22 Kohlenstoffatomen in einer Gesamtmenge von 2,0 bis 8,0 Gew.-%, bevorzugt von 2,0 bis 6,0 Gew.-% und besonders bevorzugt von 2,0 bis 4,0 Gew.-% - bezogen auf das Gewicht des Mittels - enthalten ist.

5. Mittel nach einem der Ansprüche 1 bis 4, **dadurch gekennzeichnet, dass** der mindestens eine Glycerylfettsäureester (b) der allgemeinen Formel (I) ausgewählt ist aus Glycerylmonostearat, Glyceryldistearat, Glycerylmonopalmitat und Glyceryldipalmitat sowie Mischungen hiervon.

6. Mittel nach einem der Ansprüche 1 bis 5, **dadurch gekennzeichnet, dass** der mindestens eine Glycerylfettsäureester (b) der allgemeinen Formel (I) in einer Gesamtmenge von 0,2 bis 0,6 Gew.-%, bevorzugt von 0,25 bis 0,6 Gew.-% und besonders bevorzugt von 0,3 bis 0,6 Gew.-% - bezogen auf das Gewicht des Mittels - enthalten ist.

7. Mittel nach einem der Ansprüche 1 bis 6, **dadurch gekennzeichnet, dass** es zusätzlich mindestens ein lineares gesättigtes Alkanol mit 12 - 18 Kohlenstoffatomen, insbesondere Cetylalkohol, Stearylalkohol oder Lanolinalkohol oder Gemische dieser Alkohole, wie sie bei der großtechnischen Hydrierung von pflanzlichen und tierischen Fettsäuren erhältlich sind, sowie Mischungen dieser Alkanole, enthält.

8. Mittel nach einem der Ansprüche 1 bis 7, **dadurch gekennzeichnet, dass** es zusätzlich Cetylalkohol oder Stearylalkohol oder Mischungen hiervon enthält.

9. Mittel nach einem der Ansprüche 1 bis 8, **dadurch gekennzeichnet, dass** es zusätzlich mindestens ein lineares gesättigtes Alkanol mit 12 - 18 Kohlenstoffatomen in einer Gesamtmenge von 0,1 bis 10 Gew.-%, besonders bevorzugt 1 bis 7 Gew.-%, außerordentlich bevorzugt 2 bis 5 Gew.-%, bezogen auf das Gewicht des Mittels, enthält.

10. Mittel nach einem der Ansprüche 1 bis 9, **dadurch gekennzeichnet, dass** es 50 - 80 Gew.-%, bevorzugt 60 - 75 Gew.-% Wasser, bezogen auf das Gewicht des Mittels, enthält.

11. Mittel nach einem der Ansprüche 1 bis 10, **dadurch gekennzeichnet, dass** mindestens ein ethoxylierter Fettalkohol mit einem Ethoxylierungsgrad von 80 bis 120, ausgewählt aus einer oder mehrerer Verbindungen der Formel (III) 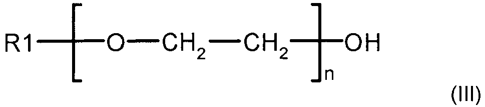
worin R1 für eine gesättigte oder ungesättigte, unverzweigte oder verzweigte C₈-C₂₄-Alkylgruppe, bevorzugt für eine gesättigte, unverzweigte C₁₆- oder C₁₈- Alkylgruppe, steht und
n für eine ganze Zahl von 80 bis 120, bevorzugt für eine ganze Zahl von 90 bis 110 und besonders bevorzugt für die Zahl 100, steht, enthalten ist.

12. Mittel nach einem der Ansprüche 1 bis 11, **dadurch gekennzeichnet, dass** mindestens ein ethoxylierter Fettalkohol mit einem Ethoxylierungsgrad von 80 bis 120, ausgewählt aus einer oder mehrerer Verbindungen der Formel (III) 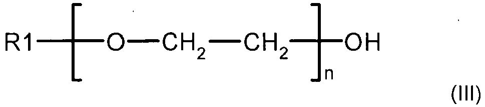
worin R1 für eine gesättigte oder ungesättigte, unverzweigte oder verzweigte C₈-C₂₄-Alkylgruppe, bevorzugt für eine gesättigte, unverzweigte C₁₆- oder C₁₈- Alkylgruppe, steht und
n für eine ganze Zahl von 80 bis 120, bevorzugt für eine ganze Zahl von 90 bis 110 und besonders bevorzugt für die Zahl 100, steht, in einer Gesamtmenge von 0,2 bis 1,5 Gew.-%, bevorzugt von 0,3 bis 1,2 Gew.-%, weiter bevorzugt von 0,4 bis 0,9 Gew.-% und besonders bevorzugt von 0,5 bis 0,8 Gew.-% - bezogen auf das Gewicht des Mittels, enthalten ist.

13. Verwendung einer Kombination aus
(a) mindestens einem linearen Alkan-1-ol oder Alken-1-ol mit jeweils 20 bis 22 Kohlenstoffatomen, wobei das mindestens eine Alken-1-ol eine bis vier olefinische Doppelbindungen aufweist, in einer Gesamtmenge von 1,8 bis 10,0 Gew.-%, bezogen auf das Gewicht des Mittels, weiter
(b) mindestens einem Glycerylfettsäureester der allgemeinen Formel (I), 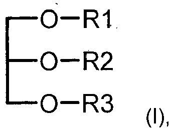 worin
R1, R2 und R3 unabhängig voneinander für ein Wasserstoffatom oder eine Gruppierung der Formel (II) stehen, 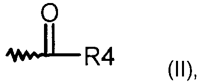 worin
R4 für eine unverzweigte oder verzweigte, gesättigte oder ungesättigte C₁₁-C₂₇₋Alkylgruppe steht, mit der Maßgabe, dass mindestens einer und maximal zwei der Reste ausgewählt aus R1, R2 und R3 für eine Gruppierung der Formel (II) stehen, und
(c) Wasser
in Mitteln zum Glätten, permanenten Umformen oder zur Depilation von Haaren, enthaltend mindestens eine Keratin reduzierende Verbindung, ausgewählt aus Thioglykolsäure und deren Salzen und/oder Thiomilchsäure und deren Salzen und/oder Cystein und/oder Acetylcystein und/oder Cysteamin und deren Salzen sowie Mischungen dieser Keratin reduzierenden Verbindungen, zur Reduktion des Geruchs der mindestens einen Keratin reduzierenden Verbindung vor, während und nach dem Glätt-, Umformungs- oder Depilationsvorgang,
**dadurch gekennzeichnet, dass** der mindestens eine Glycerylfettsäureester (b) der allgemeinen Formel (I) in einer Gesamtmenge von 0,1 bis 0,8 Gew.-% - bezogen auf das Gewicht des Mittels - enthalten ist.

14. Verwendung nach Anspruch 13 in einem Mittel gemäß einem der Ansprüche 2 bis 12.

15. Verfahren zum Glätten, permanenten Umformen oder zur Depilation von keratinischen Fasern, in welchem
(i) die Fasern unter Zuhilfenahme von mechanischen Verformungshilfsmitteln, wie Wickler oder Papilloten, nach, vor oder während des Schritts (ii) verformt werden,
(ii) ein Mittel nach einem der Ansprüche 1 bis 12 auf die Fasern aufgetragen wird,
(iii)die Fasern nach einer Einwirkzeit Z1, bevorzugt nach 5-60 Minuten, besonders bevorzugt nach 10-30 Minuten gespült und gegebenenfalls getrocknet werden,
(iv) anschließend ein Fixiermittel, enthaltend mindestens ein Oxidationsmittel, auf die Fasern aufgetragen und nach einer Einwirkzeit Z2, bevorzugt nach 1-30 Minuten, besonders bevorzugt nach 5-20 Minuten wieder abgespült wird.

## Claims

1. An agent for the straightening, permanent shaping or depilation of keratin fibers, in particular human hair, containing
(a) at least one linear alkan-1-ol or alken-1-ol in each case having 20 to 22 carbon atoms, the at least one alken-1-ol having one to four olefinic double bonds, in a total amount of 1.8 to 10.0 wt.%, based on the weight of the agent, and
(b) at least one glyceryl fatty acid ester of general formula (I), 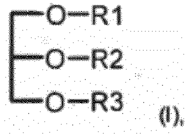 where R1, R2 and R3, independently of one another, represent a hydrogen atom or a group of formula (II), 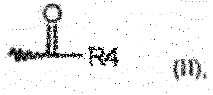 where R4 represents an unbranched or branched, saturated or unsaturated C₁₁-C₂₇ alkyl group, with the proviso that at least one and at most two of the functional groups selected from R1, R2, and R3 represent a group of formula (II), and
(c) water and
(d) at least one keratin-reducing compound, selected from thioglycolic acid and the salts thereof and/or thiolactic acid and the salts thereof and/or cysteine and/or acetylcysteine and/or cysteamine and the salts thereof, and mixtures of these keratin-reducing compounds,
**characterized in that** the at least one glyceryl fatty acid ester (b) of general formula (I) is contained in a total amount of 0.1 to 0.8 wt.%, based on the weight of the agent.

2. The agent according to claim 1, **characterized in that** the at least one keratin-reducing compound (d) is contained in a total amount of 0.1 to 20 wt.%, preferably 1 to 15 wt.%, particularly preferably 3 to 13 wt.%, in each case based on the weight of the agent.

3. The agent according to claim 1 or 2, **characterized in that** the at least one linear alkan-1-ol or alken-1-ol in each case having 20 to 22 carbon atoms is selected from arachyl alcohol (eicosan-1-ol), gadoleyl alcohol ((9*Z*)-eicos-9-en-1-ol), arachidonic alcohol ((5Z,8Z,11Z,14Z)-eicosa-5,8,11,14-tetraen-1-ol), heneicosyl alcohol (heneicosan-1-ol), behenyl alcohol (docosan-1-ol), erucyl alcohol ((13Z)-docos-13-en-1-ol), and brassidyl alcohol ((13*E*)-docosen-1-01), and mixtures thereof, and in particular is selected from mixtures of arachyl alcohol and behenyl alcohol.

4. The agent according to one of claims 1 to 3, **characterized in that** the at least one linear alkan-1-ol or alken-1-ol in each case having 20 to 22 carbon atoms is contained in a total amount of 2.0 to 8.0 wt.%, preferably 2.0 to 6.0 wt.% and particularly preferably 2.0 to 4.0 wt.%, based on the weight of the agent.

5. The agent according to one of claims 1 to 4, **characterized in that** the at least one glyceryl fatty acid ester (b) of general formula (I) is selected from glyceryl monostearate, glyceryl distearate, glyceryl monopalmitate, and glyceryl dipalmitate, and mixtures thereof.

6. The agent according to one of claims 1 to 5, **characterized in that** the at least one glyceryl fatty acid ester (b) of general formula (I) is contained in a total amount of 0.2 to 0.6 wt.%, preferably 0.25 to 0.6 wt.% and particularly preferably 0.3 to 0.6 wt.%, based on the weight of the agent.

7. The agent according to one of claims 1 to 6, **characterized in that** it additionally contains at least one linear saturated alkanol having 12-18 carbon atoms, in particular cetyl alcohol, stearyl alcohol, or lanolin alcohol, or mixtures of these alcohols, which are obtainable in the industrial-scale hydrogenation of plant and animal fatty acids, and mixtures of these alkanols.

8. The agent according to one of claims 1 to 7, **characterized in that** it additionally contains cetyl alcohol or stearyl alcohol or mixtures thereof.

9. The agent according to one of claims 1 to 8, **characterized in that** it additionally contains at least one linear saturated alkanol having 12-18 carbon atoms in a total amount of 0.1 to 10 wt.%, particularly preferably 1 to 7 wt.%, extremely preferably 2 to 5 wt.%, based on the weight of the agent.

10. The agent according to one of claims 1 to 9, **characterized in that** it contains 50-80 wt.%, preferably 60-75 wt.%, of water, based on the weight of the agent.

11. The agent according to one of claims 1 to 10, **characterized in that** at least one ethoxylated fatty alcohol having a degree of ethoxylation of 80 to 120 is contained which is selected from one or more compounds of formula (III) 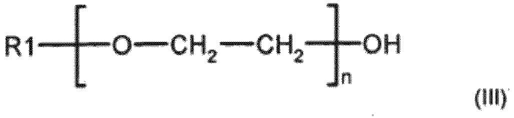 where R1 represents a saturated or unsaturated, unbranched or branched C₈-C₂₄ alkyl group, preferably a saturated, unbranched C₁₆ or C₁₈ alkyl group, and n represents an integer from 80 to 120, preferably an integer from 90 to 110, and particularly preferably the number 100.

12. The agent according to one of claims 1 to 11, **characterized in that** at least one ethoxylated fatty alcohol having a degree of ethoxylation of 80 to 120 is contained which is selected from one or more compounds of formula (III) 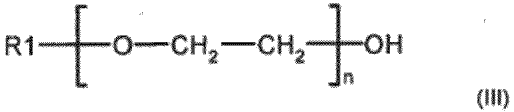 where R1 represents a saturated or unsaturated, unbranched or branched C₈-C₂₄ alkyl group, preferably a saturated, unbranched C₁₆ or C₁₈ alkyl group, and n represents an integer from 80 to 120, preferably an integer from 90 to 110 and particularly preferably the number 100, in a total amount of 0.2 to 1.5 wt.%, preferably 0.3 to 1.2 wt.%, more preferably 0.4 to 0.9 wt.% and particularly preferably 0.5 to 0.8 wt.%, based on the weight of the agent.

13. The use of a combination of
(a) at least one linear alkan-1-ol or alken-1-ol in each case having 20 to 22 carbon atoms, the at least one alken-1-ol having one to four olefinic double bonds, in a total amount of 1.8 to 10.0 wt.%, based on the weight of the agent, and
(b) at least one glyceryl fatty acid ester of general formula (I), 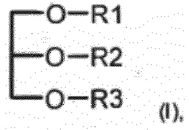 where R1, R2 and R3, independently of one another, represent a hydrogen atom or a group of formula (II), 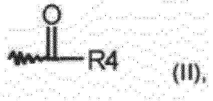 where R4 represents an unbranched or branched, saturated or unsaturated C₁₁-C₂₇ alkyl group, with the proviso that at least one and at most two of the functional groups selected from R1, R2, and R3 represent a group of formula (II), and
(c) water
in agents for the straightening, permanent shaping or depilation of hair, containing at least one keratin-reducing compound selected from thioglycolic acid and the salts thereof and/or thiolactic acid and the salts thereof and/or cysteine and/or acetylcysteine and/or cysteamine and the salts thereof, and mixtures of these keratin-reducing compounds, for reducing the odor of the at least one keratin-reducing compound before, during, and after the straightening, shaping or depilation process, **characterized in that** the at least one glyceryl fatty acid ester (b) of general formula (I) is contained in a total amount of 0.1 to 0.8 wt.%, based on the weight of the agent.

14. The use according to claim 13 in an agent according to one of claims 2 to 12.

15. A method for the straightening, permanent shaping or depilation of keratin fibers, in which
(i) the fibers are deformed with the aid of mechanical deformation means such as curlers or curlpaper after, before or during step (ii),
(ii) an agent according to one of claims 1 to 12 is applied to the fibers,
(iii) after a contact time Z1, preferably after 5-60 minutes, particularly preferably after 10-30 minutes, the fibers are rinsed and optionally dried,
(iv) a fixing agent containing at least one oxidizing agent is subsequently applied to the fibers and rinsed off after a contact time Z2, preferably after 1-30 minutes, particularly preferably after 5-20 minutes.

## Revendications

1. Agent de lissage, de mise en forme permanente ou d'épilation des fibres kératiniques, en particulier des cheveux humains, contenant
(a) au moins un alcan-1-ol ou alcén-1-ol linéaire ayant, dans chaque cas, de 20 à 22 atomes de carbone, l'au moins un alcén-1-ol présentant une à quatre doubles liaisons oléfiniques, en une quantité totale située dans la plage allant de 1,8 à 10,0 % en poids, par rapport au poids de l'agent, et
(b) au moins un ester d'acide gras glycérylique de formule générale (I), 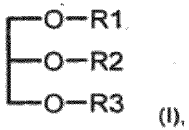 dans laquelle
R1, R2 et R3 représentent, chacun indépendamment les uns des autres, un atome d'hydrogène ou un groupe de formule (II), 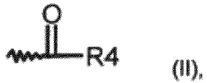 dans laquelle
R4 est un groupe alkyle en C₁₁ à C₂₇ non ramifié ou ramifié, saturé ou insaturé, à condition qu'au moins l'un et au plus deux des radicaux choisis parmi R1, R2 et R3 représentent un groupe de formule (II), et
(c) de l'eau, et
(d) au moins un composé kératoréducteur choisi parmi l'acide thioglycolique et ses sels et/ou l'acide thiolactique et ses sels et/ou la cystéine et/ou l'acétylcystéine et/ou la cystéamine et leurs sels, ainsi que les mélanges de ces composés kératoréducteurs,
**caractérisé en ce que** l'au moins un ester d'acide gras glycérylique (b) de formule générale (I) est présent en une quantité totale située dans la plage allant de 0,1 à 0,8 % en poids, par rapport au poids de l'agent.

2. Agent selon la revendication 1, **caractérisé en ce que** l'au moins un composé kératoréducteur (d) est présent en une quantité totale située dans la plage allant de 0,1 à 20 % en poids, de préférence de 1 à 15 % en poids, de manière particulièrement préférée de 3 à 13 % en poids, dans chaque cas par rapport au poids de l'agent.

3. Agent selon la revendication 1 ou 2, **caractérisée en ce que** l'au moins un alcan-1-ol ou alcén-1-ol linéaire ayant, dans chaque cas, de 20 à 22 atomes de carbone, est choisi parmi l'alcool arachylique (eicosan-1-ol), l'alcool gadoléylique ((9Z)-eicos-9-én-1-ol), l'alcool arachidonique ((5Z, 8Z, 11Z, 14Z)-eicosa-5,8,11,14-tétraén-1-ol), l'alcool hénéicosylique (hénéicosan-1-ol), l'alcool béhénylique (docosan-1-ol), l'alcool érucylique ((13Z)-docos-13-én-1-ol) et l'alcool brassidylique ((13*E*)-docosén-1-ol) et leurs mélanges, en particulier choisi parmi des mélanges d'alcool arachylique et d'alcool béhénylique.

4. Agent selon l'une des revendications 1 à 3, **caractérisé en ce que** l'au moins un alcan-1-ol ou alcén-1-ol linéaire ayant, dans chaque cas, de 20 à 22 atomes de carbone, est présent en une quantité totale située dans la plage allant de 2,0 à 8,0 % en poids, de préférence de 2,0 à 6,0 % en poids, et de manière particulièrement préférée de 2,0 à 4,0 % en poids, par rapport au poids de l'agent.

5. Agent selon l'une des revendications 1 à 4, **caractérisé en ce que** l'au moins un ester d'acide gras glycérylique (d) de formule générale (I) est choisi parmi le monostéarate de glycéryle, le distéarate de glycéryle, le monopalmitate de glycéryle et le dipalmitate de glycéryle, ainsi que leurs mélanges.

6. Agent selon l'une des revendications 1 à 5, **caractérisé en ce que** l'au moins un ester d'acide gras glycérylique (d) de formule (I) est présent en une quantité totale située dans la plage allant de 0,2 à 0,6 % en poids, de préférence de 0,25 à 0,6 % en poids, et de manière particulièrement préférée de 0,3 à 0,6 % en poids, par rapport au poids de l'agent.

7. Agent selon l'une des revendications 1 à 6, **caractérisé en ce qu'**il contient en outre au moins un alcanol linéaire saturé ayant de 12 à 18 atomes de carbone, en particulier de l'alcool cétylique, de l'alcool stéarylique ou de l'alcool de lanoline ou des mélanges de ces alcools, tels que ceux pouvant être obtenus par hydrogénation industrielle d'acides gras végétaux et animaux, ainsi que les mélanges de ces alcanols.

8. Agent selon l'une des revendications 1 à 7, **caractérisé en ce qu'**il contient en outre de l'alcool cétylique ou de l'alcool stéarylique ou des mélanges de ceux-ci.

9. Agent selon l'une des revendications 1 à 8, **caractérisé en ce qu'**il contient en outre au moins un alcanol saturé linéaire ayant de 12 à 18 atomes de carbone, en une quantité totale située dans la plage allant de 0,1 à 10 % en poids, de manière particulièrement préférée de 1 à 7 % en poids, de manière préférée entre toutes de 2 à 5 % en poids, par rapport au poids de l'agent.

10. Agent selon l'une des revendications 1 à 9, **caractérisé en ce qu'**il contient de 50 à 80 % en poids, de préférence de 60 à 75 % en poids d'eau, par rapport au poids de l'agent.

11. Agent selon l'une des revendications 1 à 10, **caractérisé en ce qu'**il contient au moins un alcool gras éthoxylé présentant un degré d'éthoxylation situé dans la plage allant de 80 à 120, choisi parmi un ou plusieurs composés de formule (III) 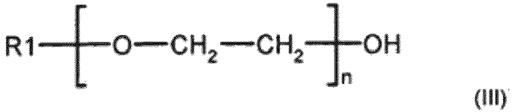 dans laquelle R1 est un groupe alkyle en C₈ à C₂₄ saturé ou insaturé, non ramifié ou ramifié, de préférence un groupe alkyle en C₁₆ ou C₁₈ saturé, non ramifié, et n représente un nombre entier situé dans la plage allant de 80 à 120, de préférence un nombre entier situé dans la plage allant de 90 à 110, et de manière particulièrement préférée le nombre 100.

12. Agent selon l'une des revendications 1 à 11, **caractérisé en ce qu'**il contient au moins un alcool gras éthoxylé présentant un degré d'éthoxylation situé dans la plage allant de 80 à 120, choisi parmi un ou plusieurs composés de formule (III) 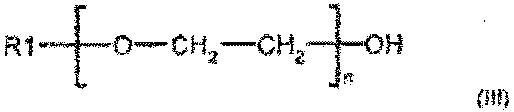 dans laquelle R1 est un groupe alkyle en C₈ à C₂₄ saturé ou insaturé, non ramifié ou ramifié, de préférence un groupe alkyle en C₁₆ ou C₁₈ saturé, non ramifié, et n représente un nombre entier situé dans la plage allant de 80 à 120, de préférence un nombre entier situé dans la plage allant de 90 à 110, et de manière particulièrement préférée le nombre 100, en une quantité totale située dans la plage allant de 0,2 à 1,5 % en poids, de préférence de 0,3 à 1,2 % en poids, de préférence encore de 0,4 à 0,9 % en poids, et de manière particulièrement préférée de 0,5 à 0,8 % en poids, par rapport au poids de l'agent.

13. Utilisation d'une combinaison
(a) d'au moins un alcan-1-ol ou alcén-1-ol linéaire ayant, dans chaque cas, de 20 à 22 atomes de carbone, dans lequel l'au moins un alcén-1-ol présente une à quatre doubles liaisons oléfiniques, en une quantité totale située dans la plage allant de 1,8 à 10,0 % en poids, par rapport au poids de l'agent, et
(b) d'au moins un ester d'acide gras glycérylique de formule générale (I), 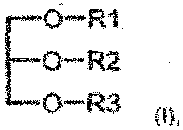 dans laquelle
R1, R2 et R3 représentent, chacun indépendamment les uns des autres, un atome d'hydrogène ou un groupe de formule (II), 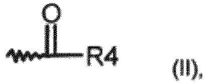 dans laquelle
R4 est un groupe alkyle en C₁₁ à C₂₇ non ramifié ou ramifié, saturé ou insaturé, à condition qu'au moins l'un et au plus deux des radicaux choisis parmi R1, R2 et R3 représentent un groupe de formule (II), et
(c) d'eau
dans des agents de lissage, de mise en forme temporaire ou d'épilation des cheveux, contenant au moins un composé kératoréducteur choisi parmi l'acide thioglycolique et ses sels et/ou l'acide thiolactique et ses sels et/ou la cystéine et/ou l'acétylcystéine et/ou la cystéamine et leurs sels, ainsi que les mélanges de ces composés kératoréducteurs, pour réduire l'odeur de l'au moins un composé kératoréducteur avant, pendant et après l'opération de lissage, de mise en forme ou d'épilation, **caractérisée en ce que** l'au moins un ester d'acide gras glycérylique (b) de formule générale (I) est présent en une quantité totale située dans la plage allant de 0,1 à 0,8 % en poids, par rapport au poids de l'agent.

14. Utilisation selon la revendication 13 dans un agent selon l'une des revendications 2 à 12.

15. Procédé de lissage, de mise en forme permanente ou d'épilation des fibres kératiniques, dans lequel
(i) les fibres sont mises en forme à l'aide de moyens mécaniques d'aide à la mise en forme, tels que des bigoudis ou des papillotes, après, avant ou pendant l'étape (ii),
(ii) un agent selon l'une des revendications 1 à 12 est appliqué sur les fibres ;
(iii) les fibres sont rincées après un temps de pose Z1, de préférence après 5 à 60 minutes, de manière particulièrement préférée après 10 à 30 minutes et, éventuellement, séchées,
(iv) un agent de fixation contenant au moins un agent oxydant, est ensuite appliqué sur les fibres et rincé après un temps de pose Z2, de préférence après 1 à 30 minutes, de manière particulièrement préférée après 5 à 20 minutes.
